# Europäisches Patentamt
# European Patent Office
## Office européen des brevets

(11) Publication number: **0 107 278**
**B1**

(12) **EUROPEAN PATENT SPECIFICATION**

(45) Date of publication of patent specification: **15.11.89**

(21) Application number: **83304487.8**

(22) Date of filing: **03.08.83**

(60) Divisional application **88116366 filed on 04.10.88.**

(51) Int. Cl.⁴: **C 12 N 15/00, C 07 H 21/04, C 12 N 1/00, G 01 N 33/86 // C12N9/50, C12R1/19, C12R1/91**

(54) **Molecular cloning of the gene for human anti-haemophilic factor IX.**

(30) Priority: **04.08.82 GB 8222485**
**06.05.83 GB 8312491**

(43) Date of publication of application:
**02.05.84 Bulletin 84/18**

(45) Publication of the grant of the patent:
**15.11.89 Bulletin 89/46**

(84) Designated Contracting States:
**AT BE CH DE FR IT LI LU NL SE**

(56) References cited:
**NATURE, vol. 299, no. 5879, 9th September 1982, pages 178-180, Macmillan Journals Ltd., K.H. CHOO et al.: "Molecular cloning of the gene for human anti-haemophilic factor IX"**
**PROC. NATL. ACAD. SCI. USA, vol. 79, no. 21, November 1982, pages 6461-6464, K. KURACHI et al.: "Isolation and characterization of a cDNA coding for human factor IX"**

(73) Proprietor: **NATIONAL RESEARCH DEVELOPMENT CORPORATION**
**101 Newington Causeway**
**London SE1 6BU (GB)**

(72) Inventor: **Brownlee, George Gow**
**1 Lathbury Road**
**Oxford OX1 7AT (GB)**
Inventor: **Choo, Kong Hong**
**127 Lower Terrace**
**San Francisco California 94114 (US)**

(74) Representative: **Percy, Richard Keith**
**Patent Department National Research Development Corporation 101 Newington Causeway.**
**London SE1 6BU (GB)**

(56) References cited:
**NUCLEIC ACIDS RESEARCH, vol. 11, no. 8, April 1983, pages 2325-2335, IRL Press Limited, Oxford, GB., M. JAYE et al.: "Isolation of a human anti-haemophilic factor IX cDNA clone using a unique 52-base synthetic oligonucleotide probe deduced from the amino acid sequence of bovine factor IX"**

Courier Press, Leamington Spa, England.

(56) References cited:
CHEMICAL ABSTRACTS, vol. 99, no. 7, 15th
August 1983, page 116, no. 48485h, Columbus,
Ohio, USA, A.L. BLOOM: "Benefits of cloning
genes for clotting factors"

PROC. NATL. ACAD. SCI. USA, vol. 76, no. 10,
October 1979, pages 4990-4994, K. KATAYAMA
et al.: "Comparison of amino acid sequence of
bovine coagulation factor IX (Christmas Factor)
with that of other vitamin K-dependent plasma
proteins"

Nucl.Ac.Res., vol. 9(4), 1981, pp. 879-894

Proc.Natl.Acad.Sci, (USA9, vol. 78 (3), 1981, pp.
1466-1470

J.Clin.Invest., vol. 61, 1978, pp. 1528-1538

Proc.Natl.Acad.Sci., (USA), vol. 77 (9), 1980, pp.
5153-5157

PROTEASES AND BIOLOGICAL CONTROL
(E.Reich ed,Cold Spring Harbor Labs. (1975),
pp. 123-149

**Description**

Background of the invention

1. Field of the invention

This invention is in the field of genetic engineering relating to factor IX DNA.

2. Description of prior art

Factor IX (Christmas factor or antihaemophilic factor B) is the zymogen of a serine protease which is required for blood coagulation via the intrinsic pathway of clotting (Jackson & Nemerson, Ann. Rev. Biochem. *49*, 765—811, 1980). This factor is synthesized in the liver and requires vitamin K for its biosynthesis (Di Scipio & Davie, Biochem. *18*, 899—904, 1979).

Human factor IX has been purified and characterised, but details of the amino acid sequence are fragmentary. It is a single-chain glycoprotein, with a molecular weight of approximately 60,000 (Suomela, Eur. J. Biochem. *71* 145—154, 1976). Like other vitamin K-dependent plasma proteins, human factor IX contains in the amino-terminal region approximately 12 gamma-carboxyglutamic acid residues (Di Scipio & Davie, Biochem. *18*, 899—904, 1979).

During the clotting process, and in the presence of $Ca^{++}$ ions, factor IX is acted upon by activated factor XI (XIa) by the cleavage of two internal peptide bonds, releasing an activation glycopeptide of 10,000 daltons (Di Scipio *et al.,* J. Clin. Invest. *61*, 1528—1538, 1978). The activated factor IX (IXa) is composed of two chains held together by at least one disulphide bond. Factor IXa then participates in the next step in the coagulation cascade by acting on factor X in the presence of activated factor VIII, $Ca^{++}$ ions, and phospholipids (Lindquist *et al.* J. Biol. Chem. *253*, 1902—1909, 1978).

Individuals deficient in factor IX (Christmas disease or haemophilia B) show bleeding symptoms which persist throughout life. Bleeding may occur spontaneously or following injury. This may take place virtually anywhere. Bleeding into the joints is common, and after repeated haemorrhages, may result in permanent and crippling deformities. The condition is a sex-linked disorder affecting males. Its frequency in the population is approximately 1 in 30,000 males.

The current method of diagnosing Christmas disease involves measurement of the titre of factor IX in plasma by a combination of a clotting assay and an immunochemical assay. Treatment of haemorrhage in the disease consists of factor IX replacement by means of intravenous transfusion of human plasma protein concentrates enriched in factor IX. The enrichment of plasma in factor IX is a time-consuming process.

A recombinant DNA method of preparing factor IX protein, if feasible, would be of interest. *Katayama et al.* Proc. Natl. Acad. Sci. *76.* 4990—4994 (1979) disclose the amino acid sequence of bovine factor IX precursor protein. At page 4990, left-hand column, *Katayama et al.* refer only to the amino-terminal sequence of the human factor IX being similar to bovine factor IX. No actual sequence information on human factor IX is given. It was a problem that factor IX mRNA has a very low abundance making it very difficult to obtain in a reasonably concentrated form by enrichment of total liver mRNA. *DiScipio et al.* Journal of Clinical Investigation, *61*, 1528—1538 (1978) disclose some human factor IX amino acid sequences, but they were all unsuitable as a basis for constructing a mixture of oligonucleotide probes or for satisfactory probing. It was therefore also a problem to find suitable probes.

3. Summary of the invention

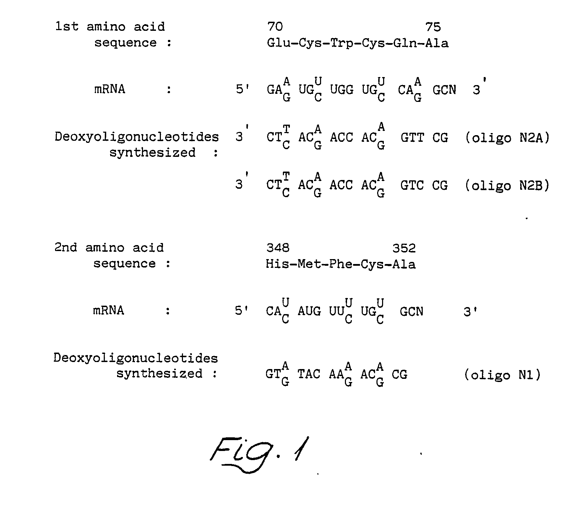

Applicants' solution is to prepare a library of bovine cDNA by using a carefully selected mixture of oligonucleotides as primer (specifically oligo N1 in Figure 1) for reverse transcriptase copying of the bovine mRNA, to use a second mixture of oligonucleotides (oligo N2A and 2B in Figure 1) as a probe to screen the bovine cDNA library, and then to use cloned *bovine* factor IX cDNA as a probe on a library of a *human* factor IX nucleic acid. As a result, applicants have prepared and sequenced DNA molecules by recombinant DNA technology which encode part or all of a precursor of human factor IX polypeptide which is convertible *in vivo* to the human factor IX polypeptide by the action of a vitamin K-dependent enzyme. A 11873 nucleotide long part of human factor IX genomic DNA sequence has been identified and a very large part of it has been sequenced by the Maxam-Gilbert sequencing method. A 129 nucleotide length of this sequence, from an exon, is more than sufficient to characterise it unambiguously as coding for a specific protein and a particular such length is regarded herein as useful to characterise the whole sequence as one occurring in the human factor IX genome. Other cloned sequences can then be verified as belonging to the human factor IX genome by determining that part thereof is identical to a region of the first-mentioned sequence, i.e. the sequences have a common identity in an overlapping region.

According to the invention therefore there is provided an artificial DNA produced by a recombinant DNA technology and either (a) encoding a precursor of human factor IX polypeptide which is convertible *in vivo* to the human factor IX polypeptide by the action of a vitamin K-dependent enzyme or (b) useful as a hybridisation probe for the diagnosis of Christmas disease, said DNA comprising (consisting of or including) substantially the following sequence of 129 nucleotides (read in rows of 30 across the page):—

ATGTAACATG    TAACATTAAG    AATGGCAGAT
GCGAGCAGTT    TTGTAAAAAT    AGTGCTGATA
ACAAGGTGGT    TTGCTCCTGT    ACTGAGGGAT
ATCGACTTGC    AGAAAACCAG    AAGTCCTGTG
AACCAGCAG

and/or its complement.

The invention includes particularly recombinant DNA which comprises a cloning vehicle DNA sequence and a sequence foreign to the cloning vehicle, wherein the foreign sequence includes substantially the whole of the above sequence.

The intron sequences of the human factor IX genome are excised during the transcription process by which mRNA is made in human cells. Only exon sequences are translated into protein. DNA coding for factor IX has been prepared from human mRNA. This cDNA has been partly sequenced and found to contain the same 129-nucleotide sequence as set out above.

The invention also includes specifically recombinant DNA which comprises a cloning vehicle sequence and a DNA sequence foreign to the cloning vehicle, wherein the foreign sequence comprises a DNA sequence which is complementary to human factor IX mRNA. Such a recombinant cDNA can be isolated from a library of recombinant cDNA clones derived from human liver mRNA by using an exon of the genomic human factor IX DNA (or part thereof) as a probe to screen this library and thence isolating the resulting clones.

Methods of isolating recombinant DNA from clones are well known and some are described hereinafter. The DNA of the invention can be single or double stranded form.

The recombinamt human factor IX DNA of this invention is useful as a tool of recombinant DNA technology. Thus it is useful as the first stage of the production of artificial human factor IX and in the preparation of probes for diagnostic purposes.

In the production of artificial human factor IX it is contemplated that appropriate cDNA or genomic clones will be introduced into a suitable expression vector in either mammalian or bacterial systems. For mammalian studies, the gene might be too long to be conveniently retained in one clone. Therefore a suitable artificial "minigene" will be designed and constructed from suitable parts of the cDNA and genomic clones. The minigene will be under the control of its own promoter or instead will be replaced by an artificial one, perhaps the mouse metallothioneine I promoter. The resultant 'minigene' will then be introduced into mammalian tissue culture cells e.g. a hepatoma cell line, and selection for clones of cells synthesising maximum amounts of biologically active factor IX will be carried out. Alternatively "genetic farming" could be employed as has been demonstrated for mouse growth hormone (Palmiter *et al.,* Nature *300*, 611—615, 1982). The minigene would be micro-injected into the pronucleus of fertilised eggs, followed by *in vivo* cloning and selection for progeny producing the largest quantity of human factor IX in blood. Alternatively, it is contemplated that the cDNA clone or selected parts of it will be linked to a suitable strong bacterial promoter, e.g. a *Lac* or *Trp* promoter or the lambda $P_R$ or $P_L$, and a factor IX polypeptide obtained therefrom.

The natural factor IX polypeptide is synthesized as a precursor containing both a signal and propeptide region. They are both normally cleaved off in the production of the definitive length protein. Even this product is merely a precursor. It is biologically inactive and must be gamma-carboxylated at 12 specific N-terminal glutamic acid residues in the so called 'GLA' domain by the action of a specific vitamin K-dependent carboxylase. In addition, two carbohydrate molecules are added to the connecting peptide region of the molecule, but it remains unknown whether they are required for activity. The substrate for the carboxylase is unknown and could be the precursor factor IX polypeptide or alternatively the definitive length protein. Therefore various relevant polypeptides both with and without the precursor domains will be "constructed" using genetic engineering methods in bacterial hosts. They will then be tested as substrates for the conversion of inactive to biologically active factor IX *in vitro* by the action of partially purified preparations of the carboxylase enzyme which can be isolated from liver microsomes or other suitable sources.

For diagnostic purposes, the recombinant human genomic factor IX DNA or recombinant human mRNA-derived factor IX DNA has a wide variety of uses. It can be cleaved by enzymes or combinations of two or more enzymes into shorter fragments of DNA which can be recombined into the cloning vehicle, producing 'sub-clones". These sub-clones can themselves be cleaved by restriction enzymes to DNA molecules suitable for preparing probes. A probe DNA (by definition) is labelled in some way, conveniently radiolabelled, and can be used to examine in detail mutations in the human DNA which ordinarily would produce factor IX. Several different probes have been produced for examining several different regions of the genome where mutation was suspected to have occurred in patients. Failure to obtain hybridisation from such a probe indicates that the sequence of the probe differs in the patient's DNA. In particular it has been shown that Christmas disease can be detected or confirmed by such methodology. Useful probes can contain intron and/or exon regions of the genomic DNA or can contain cDNA derived from the mRNA.

The invention includes particularly probe DNA, i.e., which is labelled, and of a length suitable for the probing use envisaged. It can be single-stranded or double-stranded over at least the human factor IX DNA probing sequences thereof and such sequences will not usually be larger than 5 kb and rarely longer than 10 kb.

The invention accordingly includes a DNA molecule, comprising part of the human factor IX DNA sequence as defined above whether or not labelled, whether intron or exon or partly both. It also includes human cDNA corresponding to said part or all of human factor IX mRNA. It includes particularly a solution of any DNA of the invention, which is a form in which it is conveniently obtainable by electroelution from a gel.

The invention includes, of course, a host transformed with any of the recombinant DNA of the invention. The host can be a bacterium, for example an appropriate strain of E. coli, chosen according to the nature of the cloning vehicle employed. Useful hosts may include strains of Pseudomonas, Bacillus subtilis and Bacillus stearothermophilus, other Bacilli, yeasts and other fungi and mammalian (including human) cells.

One process practised in connection with this invention for preparing a host transformed with the recombinant DNA of the invention is based on the following steps:—

(1) synthesising an oligodeoxynucleotide having a nucleotide sequence comprising that occurring in bovine factor IX messenger RNA coding for amino acids 70—75 or 348—352 of bovine factor IX, and labelling the oligodeoxynucleotide to form a probe;

(2) preparing complementary DNA to a mixture of bovine mRNAs;

(3) inserting the complementary DNA in a cloning vector to form a mixture of recombinant bovine cDNAs;

(4) transforming a host with said mixture of recombinant bovine cDNAs to form a library of clones and multiplying said clones;

(5) probing the clones with the synthetic oligodeoxynucleotide probe obtained in step 1 and isolating the resultant recombinant bovine factor IX cDNA-containing clone;

(6) digesting the recombinant bovine factor IX cDNA from said clone with one or more enzymes to produce a bovine factor IX cDNA molecule comprising a shorter sequence of bovine factor IX DNA, but preferably at least 50 base-pairs long; and

(7) probing a library of recombinant human genomic DNA in a transformed host with the shorter sequence bovine factor IX cDNA molecule, to hybridise the human genomic DNA to the said recombinant bovine factor IX DNA and isolating the resultant recombinant DNA-transformed host.

Brief description of the drawings

Figure 1 shows the structure of a published amino-acid sequence of bovine factor IX polypeptide, the deduced sequence of the mRNA from which it would be translated and the structures of oligonucleotides (oligo-N1 and N2) synthesised in the course of this invention;

Figures 2 and 3 show the chemical formulae of "building blocks" used to synthesise the oligonucleotides referred to in Figures 1 and 11;

Figure 4 is an elevational view, partly sectioned, showing an apparatus for synthesising oligonucleotides;

Figure 5 shows the sequence of part of the bovine factor IX cDNA obtained in this invention;

Figure 6 is a map showing the organisation of an approximately 27 kb length of human factor IX genomic DNA and is divided into five portions, showing:—

(a) the exon regions;

(b) the 11,873-nucleotide length sequenced;

(c) cDNA molecules obtained by restriction with various endonucleases, sub-cloned and subsequently used as probes;

(d) DNA molecules obtained by restriction with various endonucleases; and

(e) three regions of human factor IX genomic DNA derived from three clones in lambda phage vector.

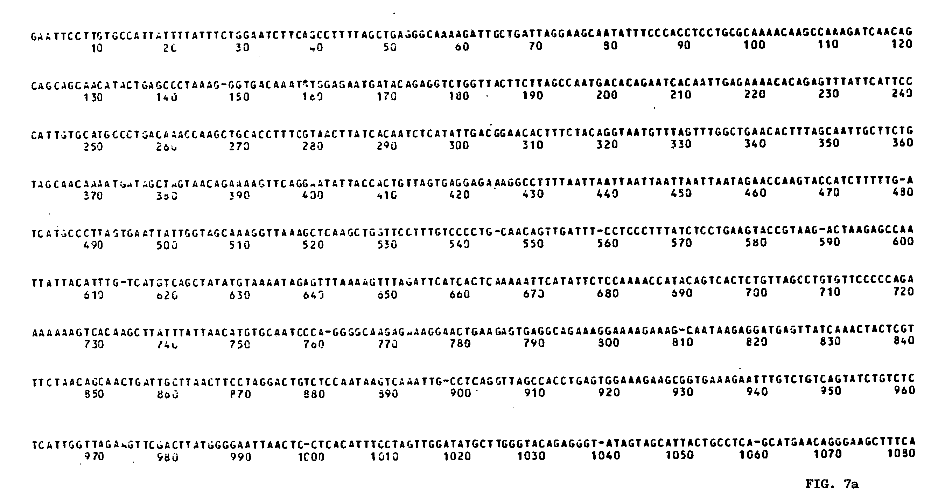

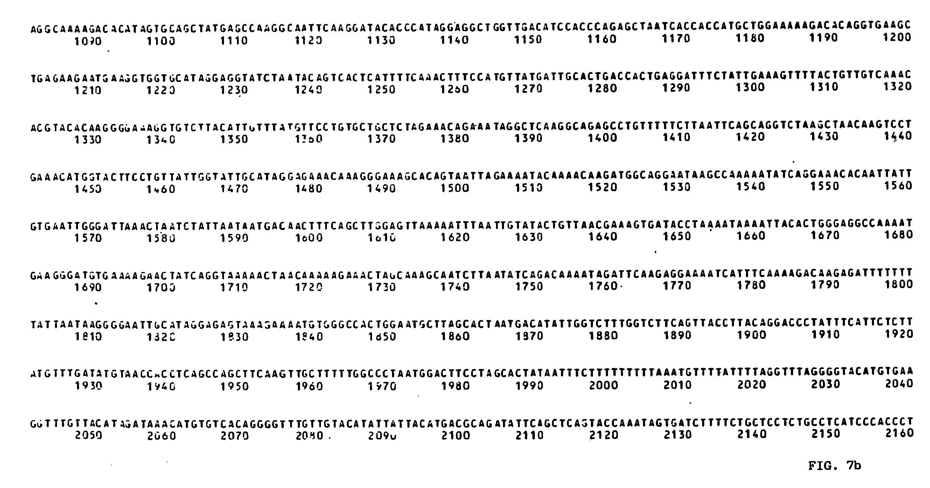

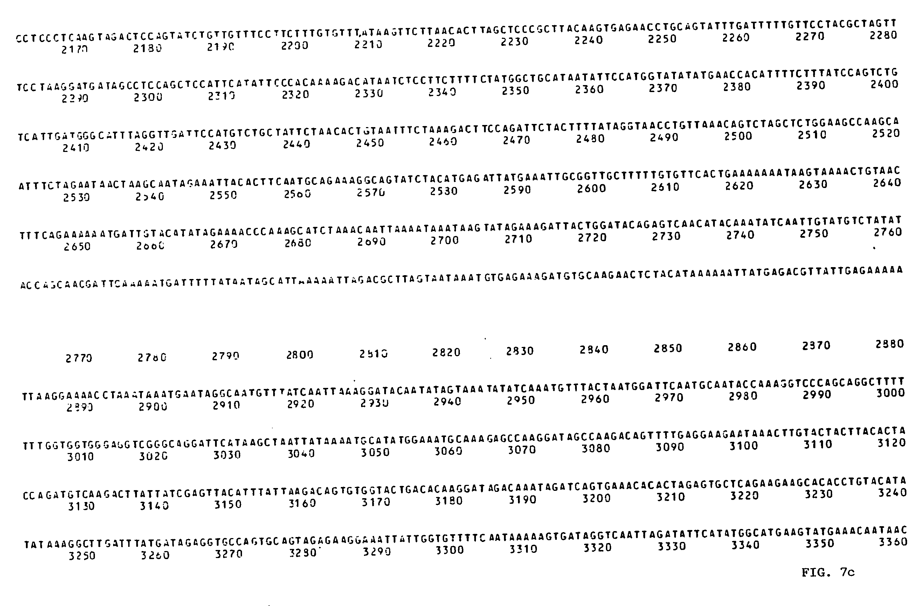

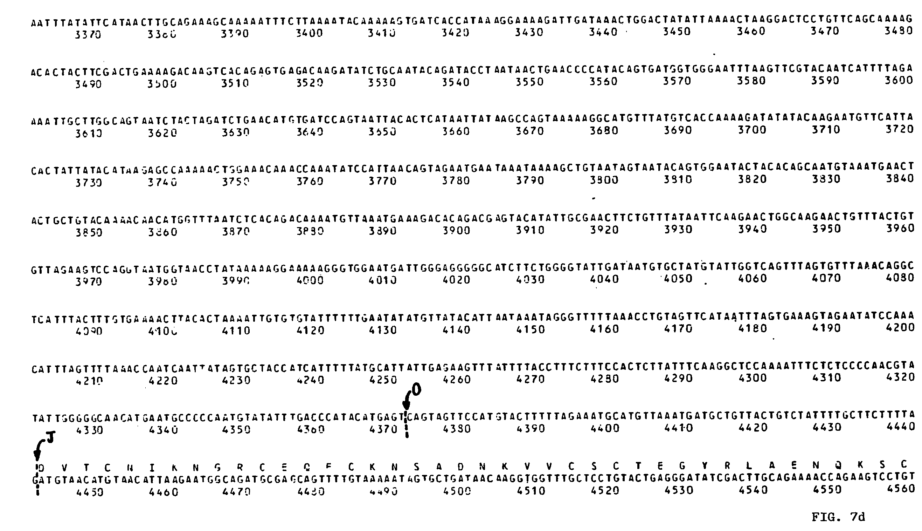

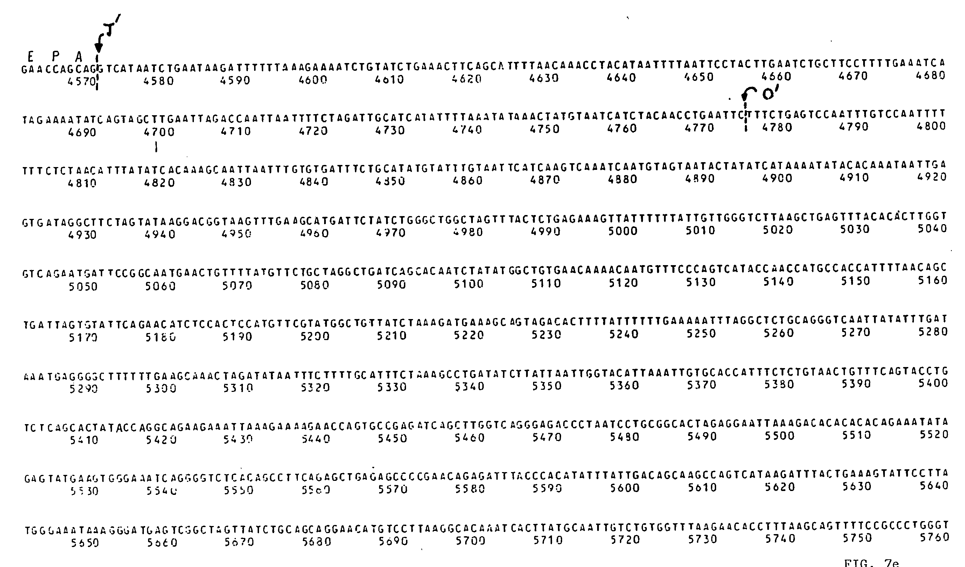

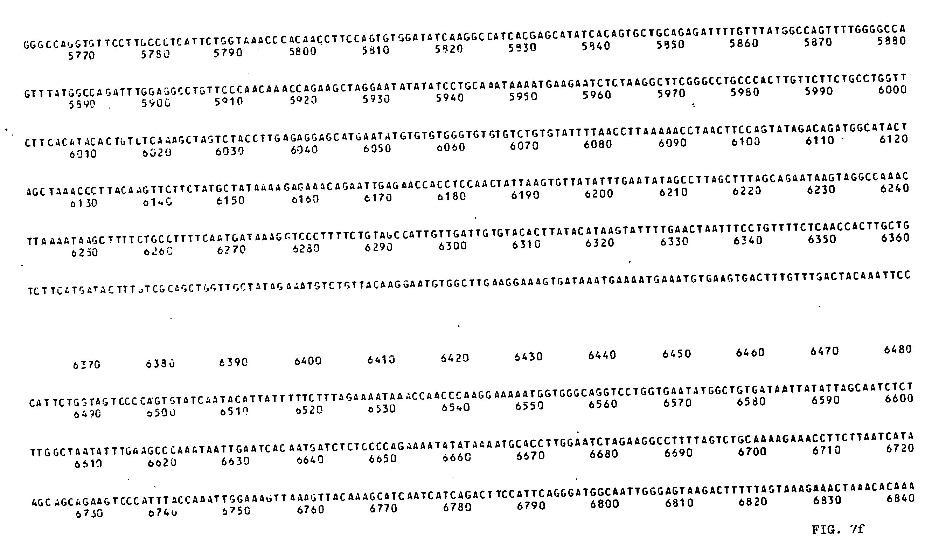

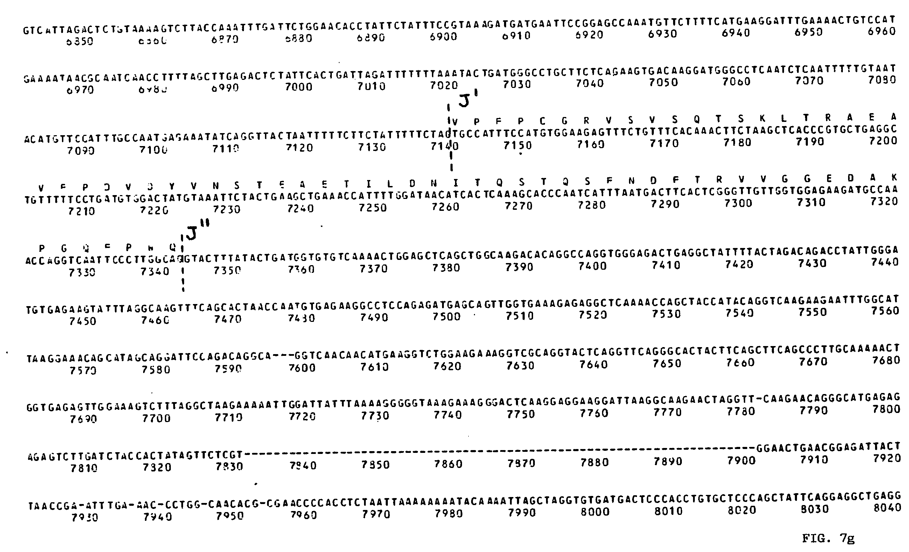

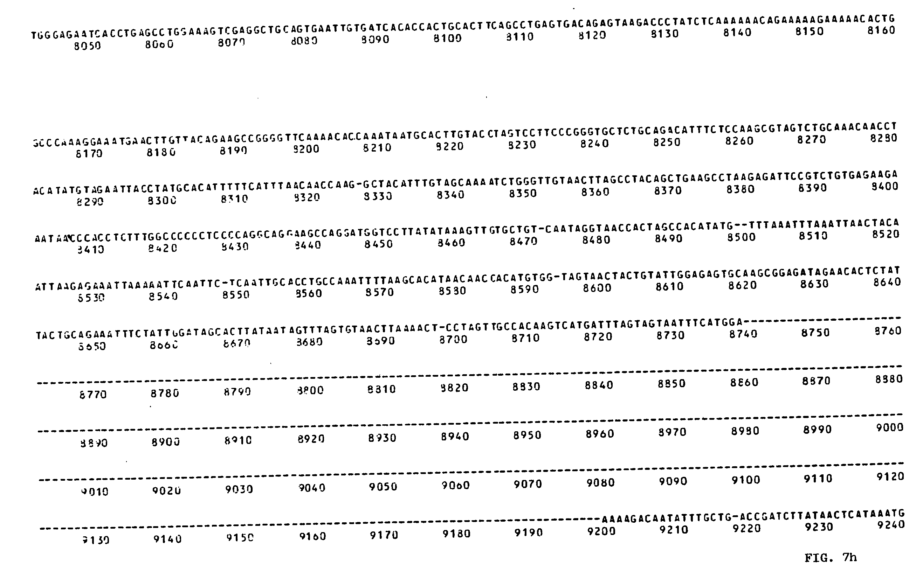

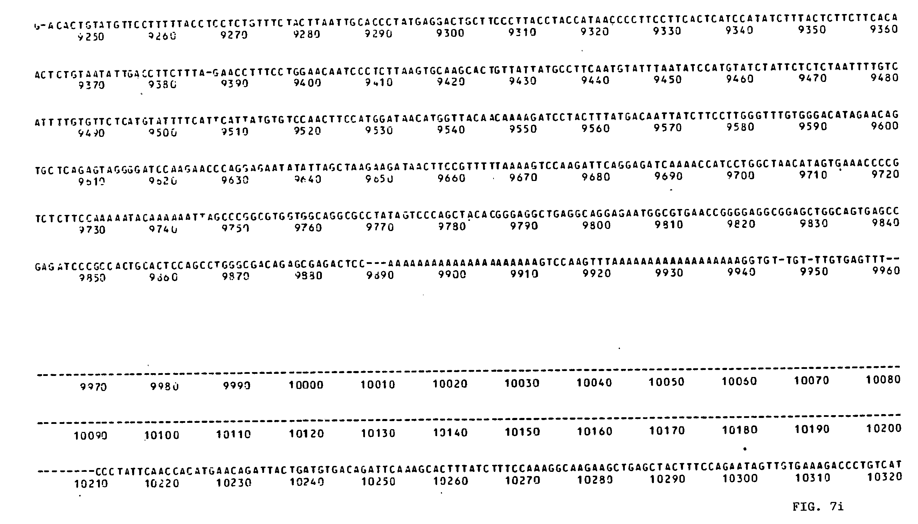

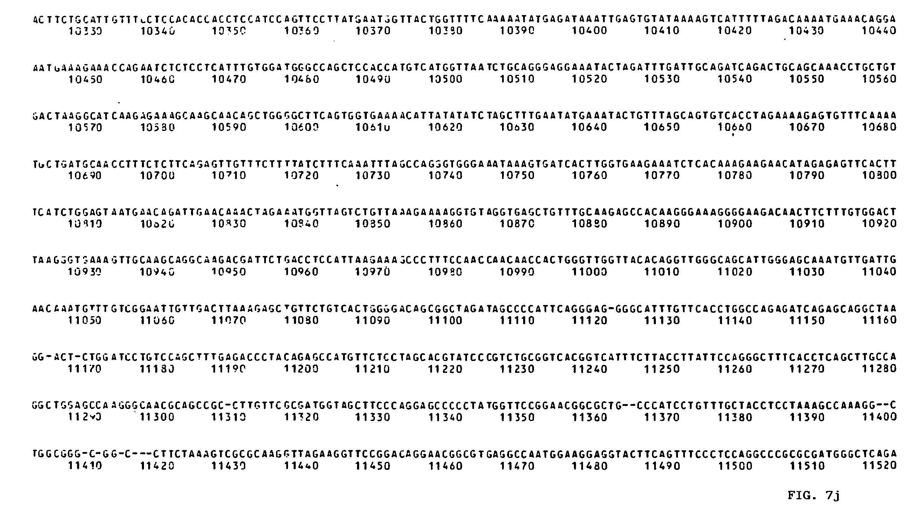

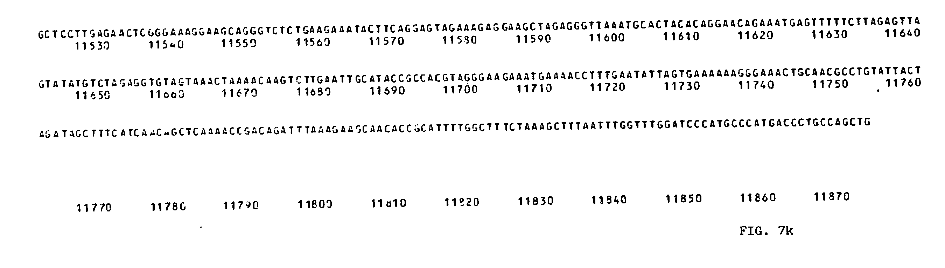

Figure 7 shows the sequence of the DNA of Figure 6(b) and in parts the encoded protein;

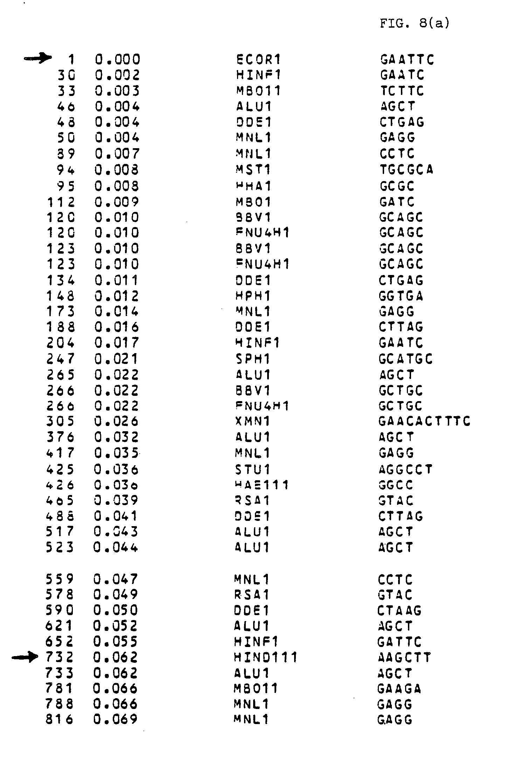

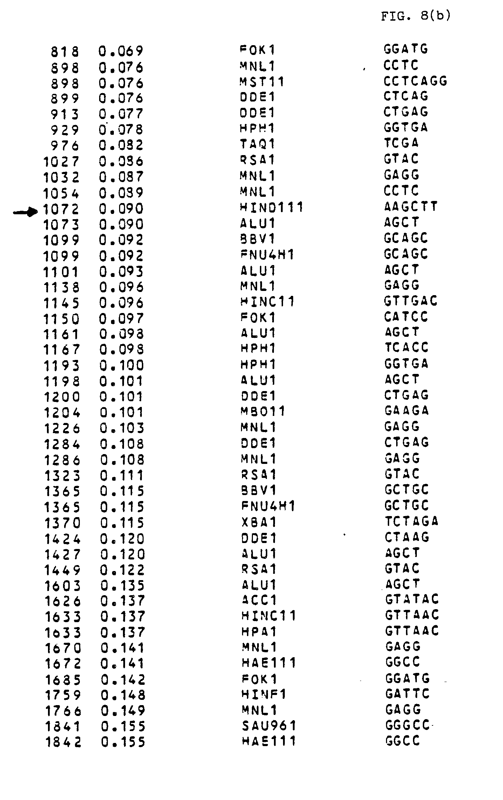

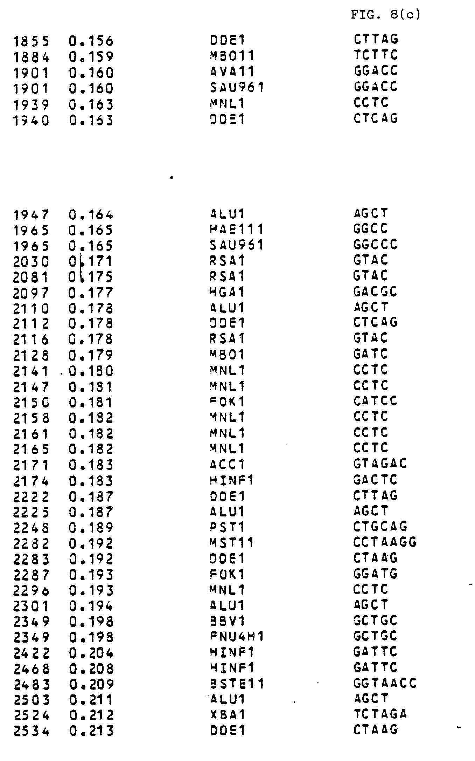

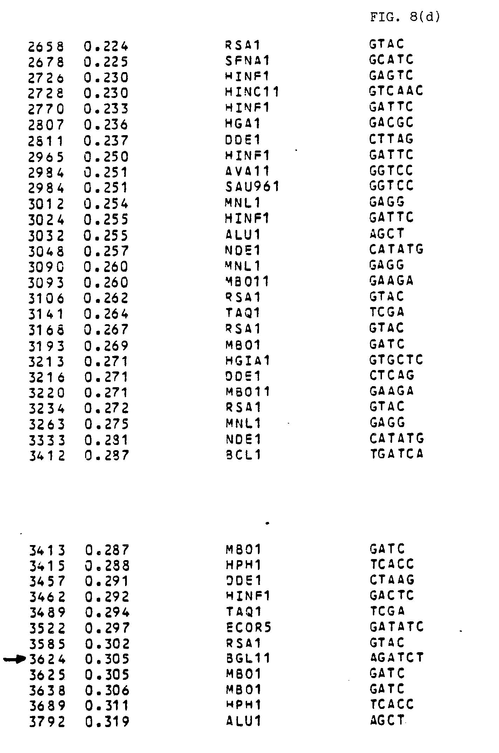

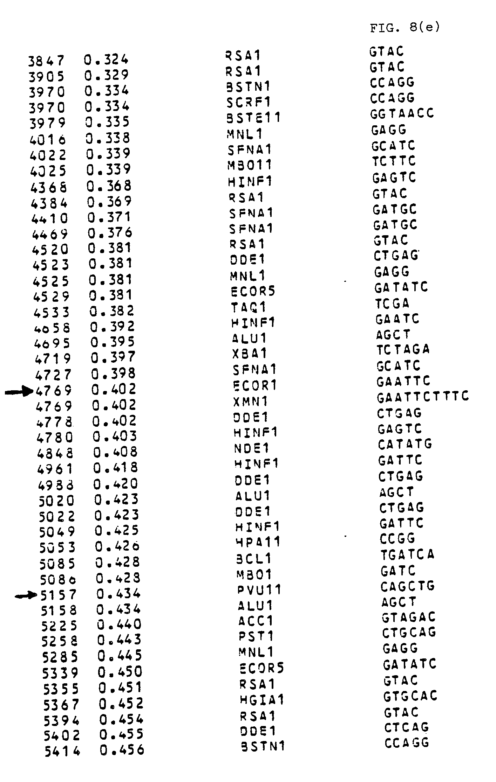

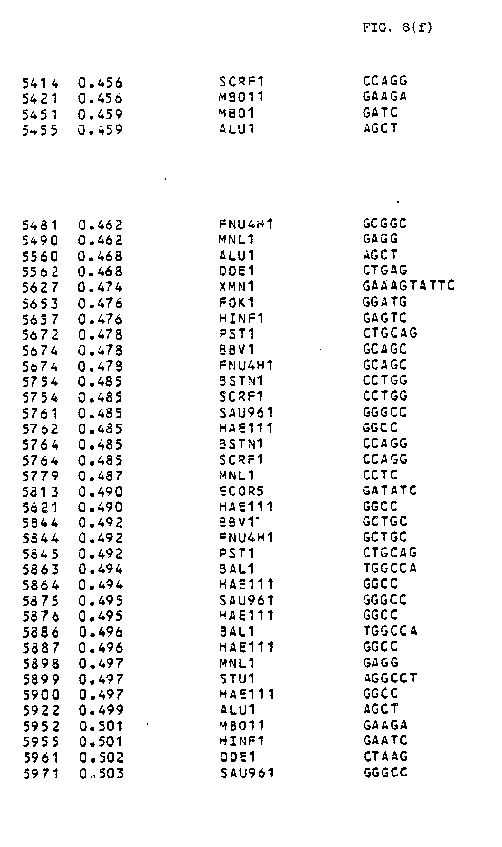

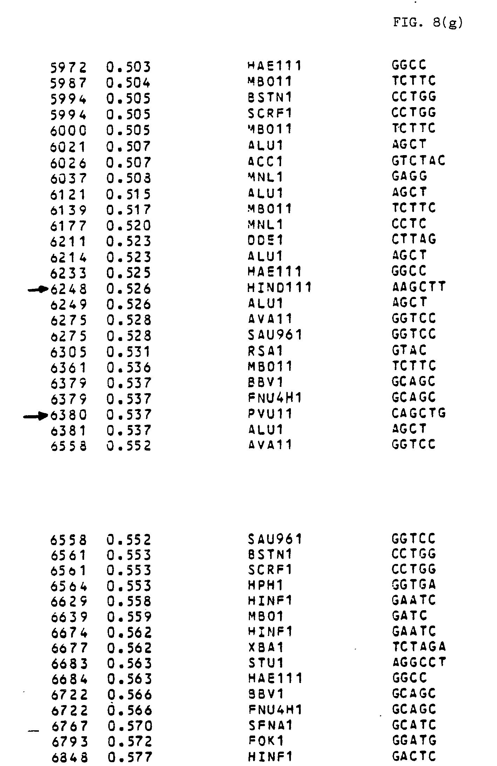

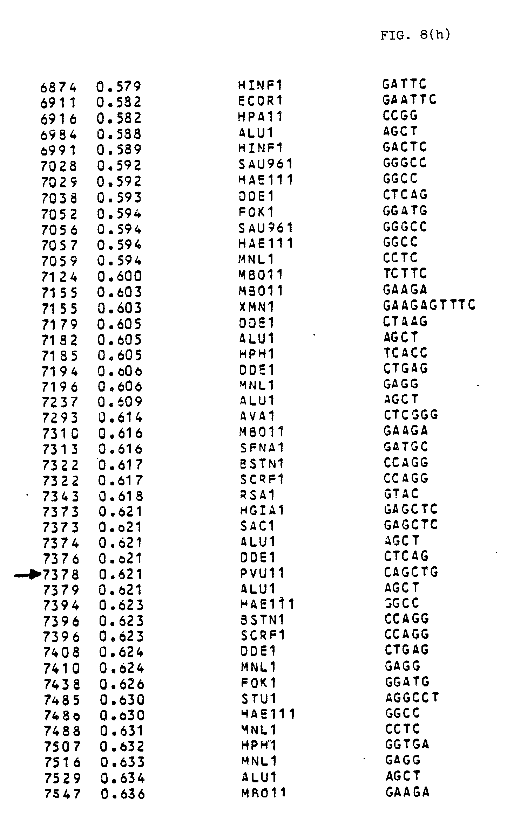

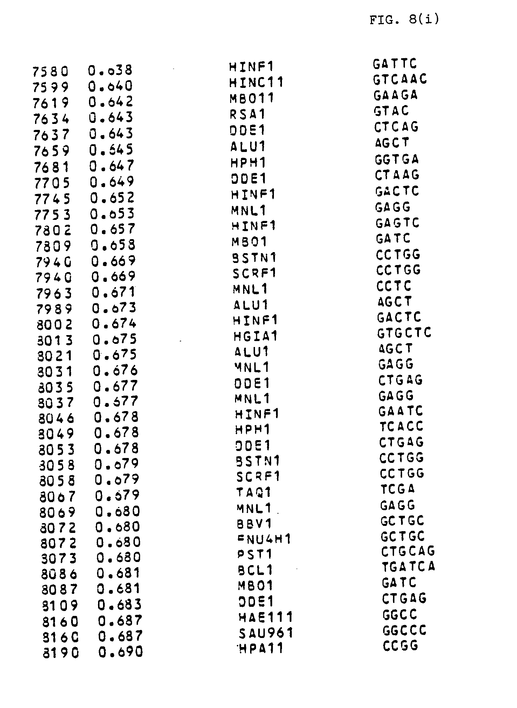

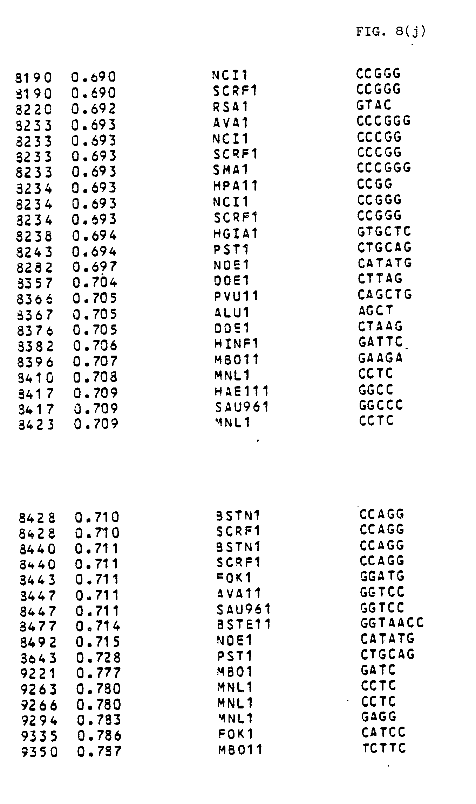

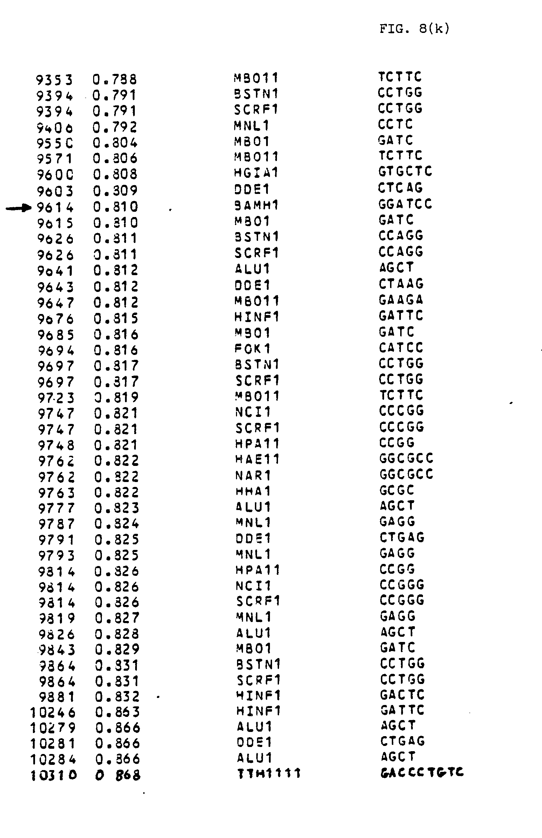

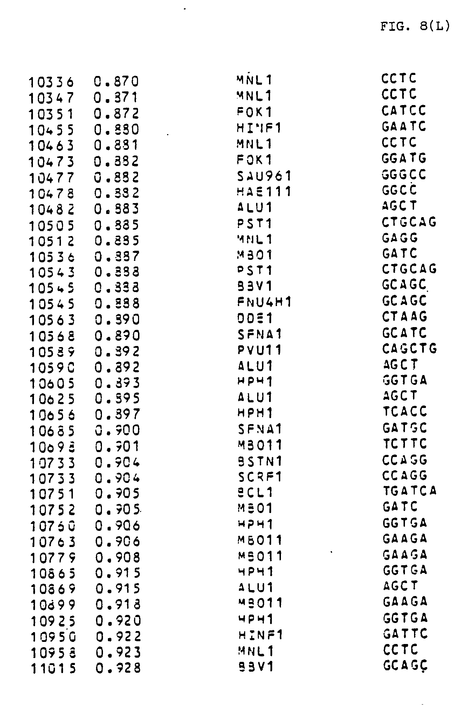

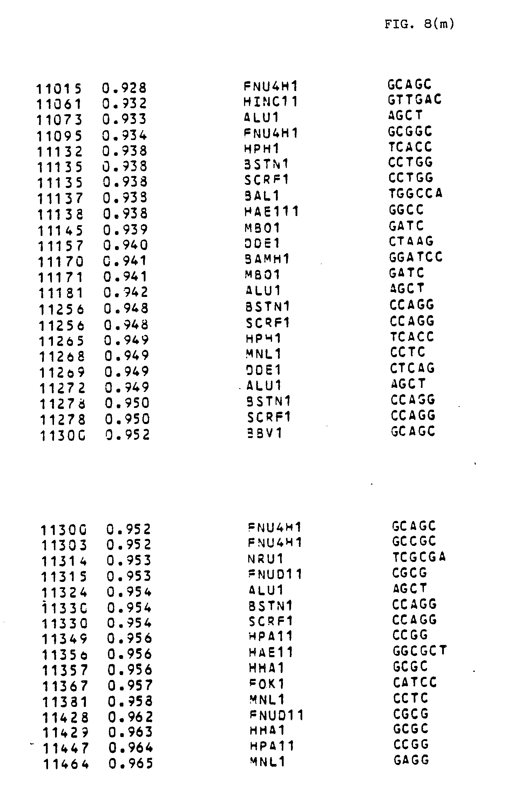

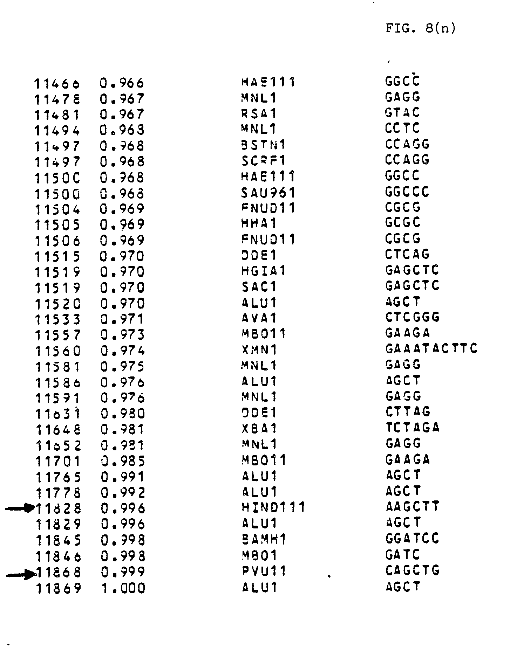

Figure 8 shows a restriction enzyme chart of the sequence shown in Figure 7;

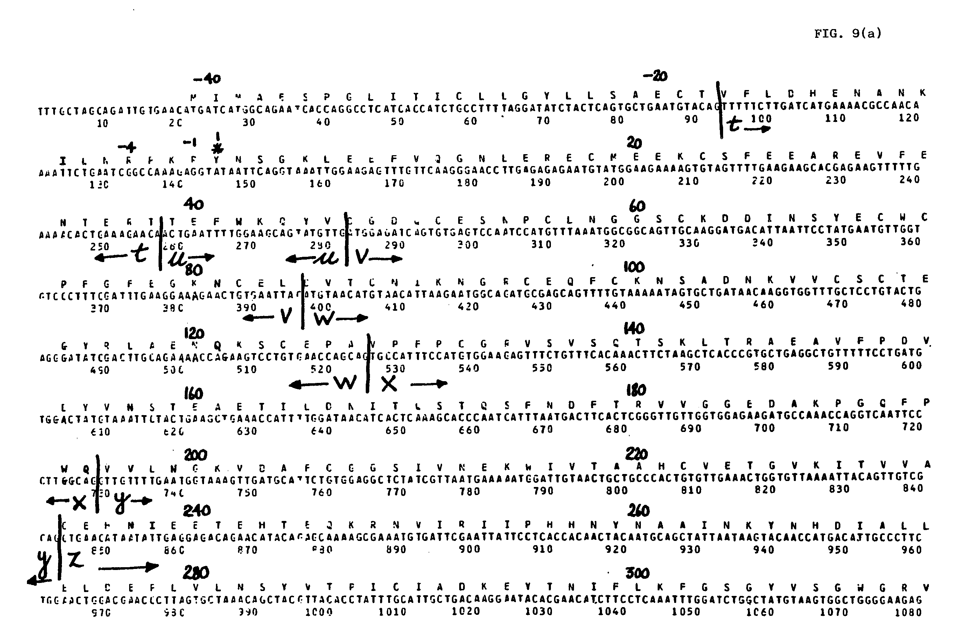

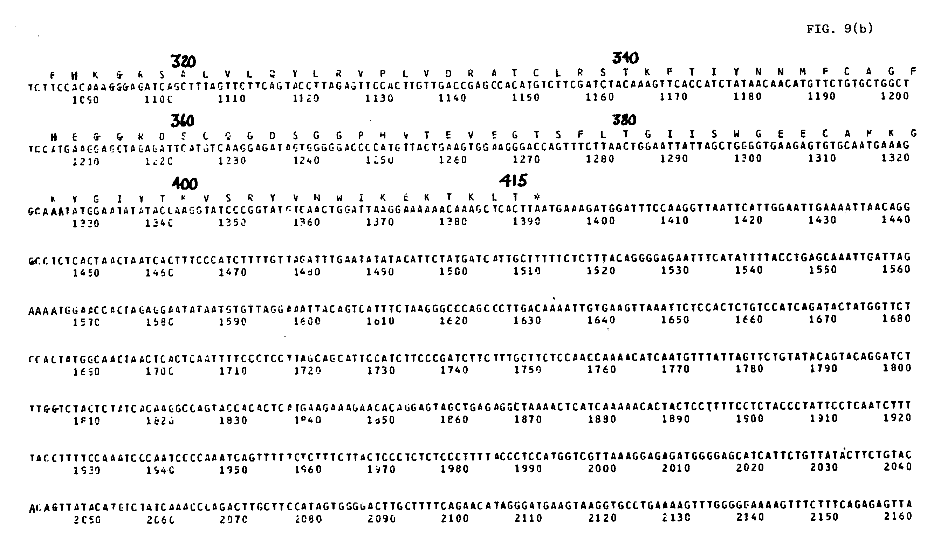

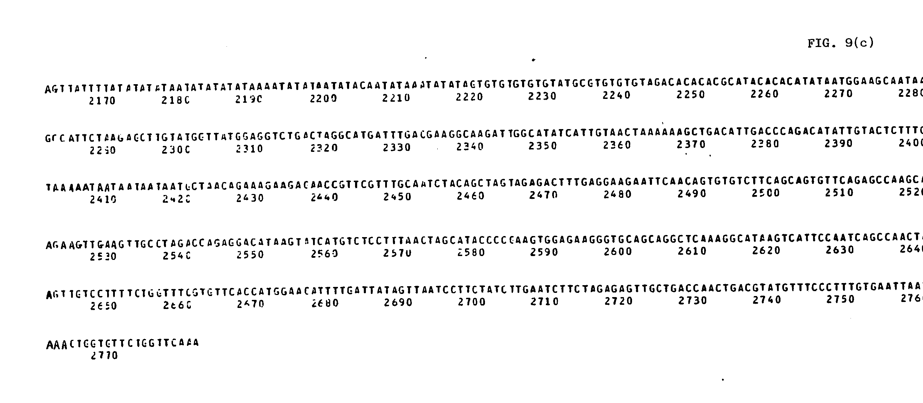

Figure 9 shows part of the sequence of the human factor IX cDNA and its encoded protein;

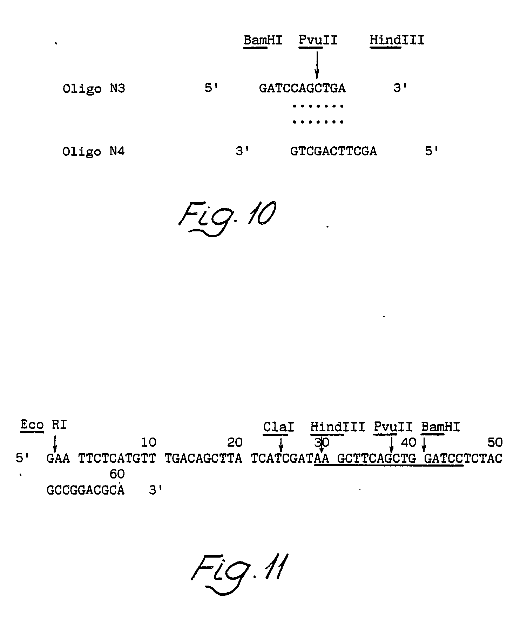

Figure 10 shows the structure of a pair of complementary oligonucleotides (oligo N3 and N4) synthesised in the course of this invention;

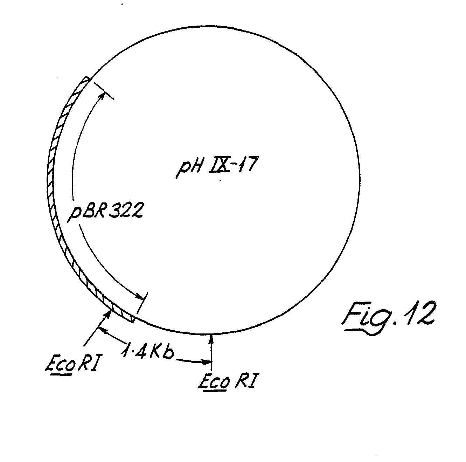

Figure 11 shows part of the DNA sequence of the vector pAT153/PvuII/8 of this invention, in the region where it differs from pAT153;

Figure 12 is a diagram of plasmid pHIX17 of the invention showing the origin of the 1.4 kb fragment used for probing and initial sequencing; and

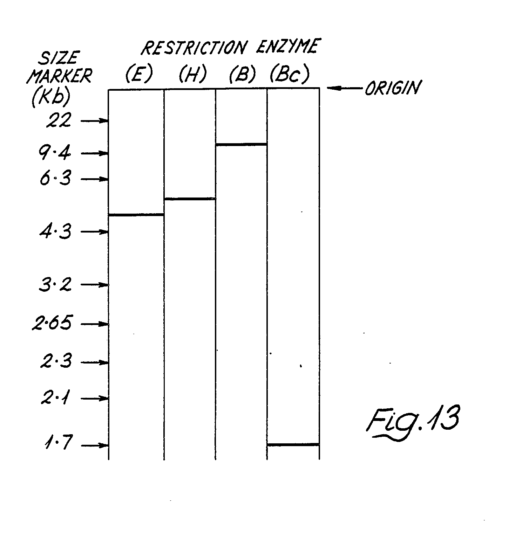

Figure 13 shows the position of the major radioactive bands on probing a "Southern blot" of normal human DNA, cut by the restriction enzymes EcoRI(E), HindIII(H), BgIII(B) and BcII(Bc), with a sub-clone of the recombinant human factor IX DNA of this invention.

Description of preferred embodiments

1. General description

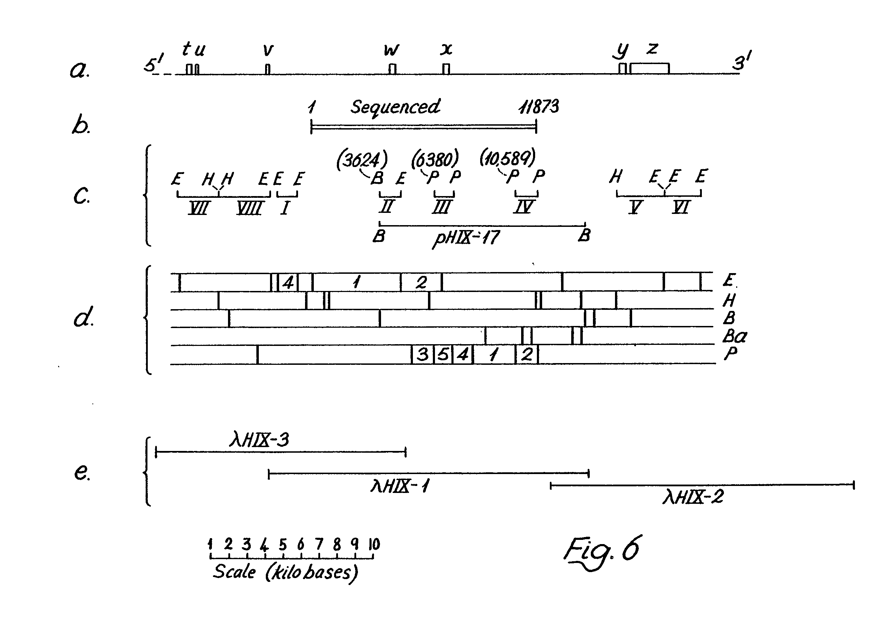

A recombinant DNA of the invention can be extracted by means of probes from a library of cloned human genomic DNA. This is a known recombinant library and the invention does not, of course, extend to human genomic factor IX DNA when present in such a library. The probes used were of bovine factor IX cDNA (DNA complementary to bovine mRNA), which were prepared by an elaborate process involving firstly the preparation of recombinant bovine cDNA from a bovine mRNA starting material, secondly the chemical syntheses of oligonucleotides, thirdly their use to probe the recombinant bovine cDNA, in order to extract bovine factor IX cDNA and fourthly the preparation of suitable probes of shorter length from the recombinant bovine factor IX cDNA. The first probe tried appeared to contain an irrelevant sequence and the second probe tried, not containing it, proved successful in enabling a single clone of the human genomic factor IX DNA to be isolated. This clone is designated lambda HIX-1. The steps involved are described in more detail in the sub-section "Examples" appearing hereinafter, and the second probe comprises the 247 base-pair DNA sequence of bovine factor IX cDNA indicated in Figure 5 of the drawings. The invention therefore provides specifically a recombinant DNA which comprises a cloning vehicle sequence and a DNA sequence foreign to the cloning vehicle, which recombinant DNA hybridises to a 247 base-pair sequence of bovine factor IX cDNA indicated in Figure 5 (by the arrows at each end thereof).

The cloning vehicle or vector employed in the invention can be any of those known in the genetic engineering art (but will be chosen to be compatible with the host). They include E. coli. plasmids, e.g. pBR322, pAT153 and modifications thereof, plasmids with wider host ranges, e.g. RP4 plasmids specific to other bacterial hosts, phages, especially lambda phage, and cosmids. A cosmid cloning vehicle contains a fragment of phage DNA including its cos (cohesive-end site) inserted in a plasmid. The resultant recombinant DNA is circular and has the capacity to accommodate very large fragments of additional foreign DNA.

Fragments of human factor IX genomic DNA can be prepared by digesting the cloned DNA with various restriction enzymes. If desired, the fragments can be religated to a cloning vehicle to prepare further recombinant DNA and thereby obtain "sub-clones". In connection with this embodiment a new cloning vehicle has been prepared. This is a modified pAT153 plasmid prepared by ligating a BamHI and HindIII double digest of pAT153 to a pair of complementary double sticky-ended oligonucleotides having a DNA sequence providing a BamHI restriction residue at one end, a HindIII restriction residue at the other end and a PvuII restriction site in between.

While the invention is described herein with reference to human genomic factor IX DNA in particular, the invention includes human factor IX cDNA (complementary to human factor IX mRNA) which contains substantially the same sequences. A library of human cDNA has been prepared and probed with human factor IX genomic DNA to isolate human factor IX cDNA from the library. For this purpose the probe DNA is conveniently of relatively short length and must include at least one exon sequence. The invention therefore includes a process of preparing a host transformed with recombinant DNA, comprising cloning vector sequences and a sequence of nucleotides comprised in cDNA complementary to human factor IX mRNA, which process comprises probing a library of clones containing recombinant DNA complementary to human mRNA with a probe comprising a labelled DNA comprising a sequence as defined above.

2. Examples
A. Bacteria used

E. coli K-12 strain MC 1061 (Casadaban & Cohen, J. Mol. Biol. *138*, 179—207, 1980). E. coli K-12 strain HB 101 (Boyer & Roulland-Dussoix, J. Mol. Biol. *41*, 459—472, 1969) and E. coli K-12 strain K803 which is a known strain used by genetic engineers.

B. Source and purification of bovine factor IX, anti-bovine factor IX antibody, and bovine mRNA

Highly purified bovine factor IX and rabbit anti-bovine factor IX antiserum were gifts from Dr. M. P. Esnouf. Analysis of the purified bovine factor IX on a denaturing polyacrylamide gel showed that it has a purity of greater than 99%. Specific anti-factor IX immunoglobulins used for immunoprecipitation experiments were purified as described by Choo et al., Biochem. J. *199*, 527—535, 1981, by passage of the crude antiserum through a Sepharose-4B column onto which pure bovine factor IX has been coupled.

Bovine mRNA was obtained from calf liver and isolated by the guanidine hydrochloride method (Chirgwin et al., Biochem, *18*, 5294—5299, 1979). The mRNA preparation was passaged through an oligo dT-cellulose column (Caton and Robertson, Nucl. Acids, Res. *7*, 1445—1456, 1979) to isolate poly-(A)+mRNA. Poly(A)+mRNA was translated in a rabbit reticulocyte cell-free system in the presence of $^{35}$S-cysteine as described by Pelham and Jackson (Eur. J. Biochem. *67*, 247—256, 1976). At the end of the translation reaction, factor IX polypeptide was precipitated by the addition of specific anti-factor IX immunoglobulins. The immunoprecipitation procedure was as described by Choo et al., Biochem. J. *181*, 285—294, 1979. The immunoprecipitated material was washed throughly and resolved on a two-dimensional SDS-polyacrylamide gel (Choo et al., Biochem. J. *181*, 285—294, 1979), by isoelectric focussing in one dimension and electrophoresis in another. Some polypeptides of known molecular weight were subjected to this procedure, to serve as reference points. The immunoprecipitated material showed 4 pronounced spots, all in the 50,000 molecular weight region and with separated isoelectric points. These predominant spots of molecular weight about 50,000 represent a single polypeptide chain plus a possible prepeptide signal sequence, a deduction compatible with published data (Katayama et al., Proc. Natl. Acad. Sci. USA *76*, 4990—4994, 1979).

When the gel analysis was repeated for the same material but immunoprecipitated in the presence of unlabelled pure bovine factor IX, the 4 spots appeared at reduced intensity, indicating that the translation product is specifically competed for by pure factor IX. Thirdly, immunoprecipitation was performed using a control rabbit antiserum, i.e. from a rabbit which had not been immunised with factor IX. None of the 4 spots appeared. These results therefore indicate that the translation product was a factor IX polypeptide.

The specific immunological/cell-free translation assay established above was used to monitor the enrichment of factor IX mRNA on sucrose gradient centrifugations. Total poly(A)+mRNA was resolved by two successive separations by sucrose gradient centrifugations. When individual fractions from the gradient were assayed by the above method, a fraction of size 20—22 Svedberg units (approx. 2.5 kilobases of RNA) region was found to be enriched (approx. ten-fold) for the bovine factor IX mRNA. This enriched fraction was used in the subsequent cloning experiments.

C. Synthesis of specific bovine factor IX deoxyoligonucleotide mixtures

Starting from a knowledge of the amino acid sequence of bovine factor IX (Katayama *et al.*, Proc. Natl. Acad. Sci. USA· *76*, 4990—4994, 1979), the synthesis of two mixtures of oligonucleotide probes was designed. These probes consisted of DNA sequences coding for two different regions of the protein. The regions selected were those known to differ in sequence in the analogous serine proteases, prothrombin, Factor C and Factors VII and X and were those corresponding to amino acids 70—75 and 348—352 respectively. The 70—75 region was particularly favourable in that the mixture of oligonucleotides synthesised, i.e. oligo N2A and oligo N2B, contained all 16 possible sequences that might occur in a 17 nucleotide long region of the mRNA corresponding to amino acids 70—75. The oligo N2A—N2B mixture is hereinafter called "olilgo N2" for brevity.

Figure 1 of the drawings shows the two selected regions of the known amino acid sequence of bovine factor IX, the corresponding mRNA and the oligonucleotides synthesised. Since some of the amino acids are coded for by more than one nucleotide triplet, there are 4 ambiguities in the mRNA sequence shown for amino acids 70—75 and therefore 16 possible individual sequences.

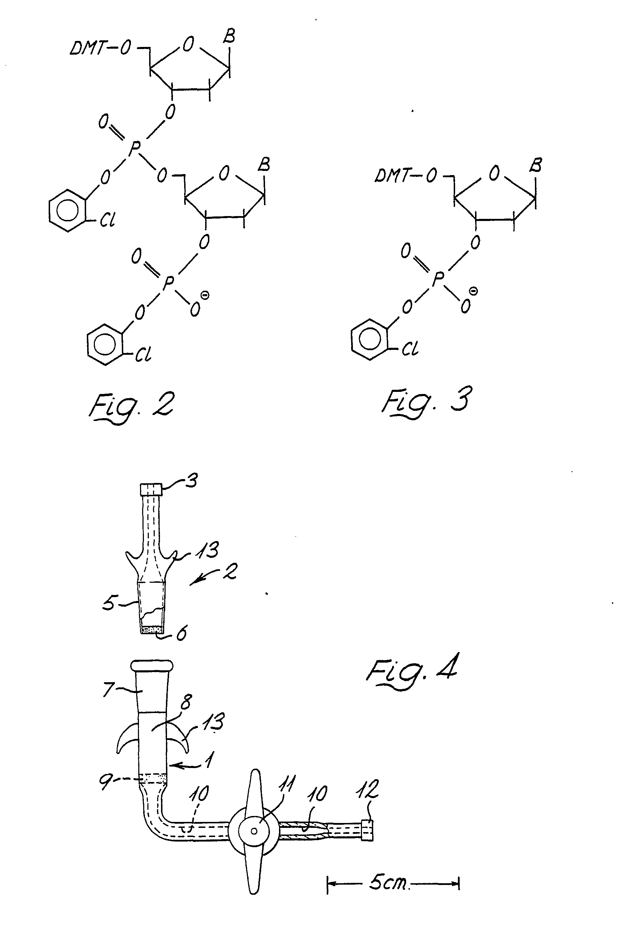

The nucleotide mixtures oligo N1 and oligo N2 were synthesized using the solid phase phosphotriester method of Duckworth *et al.*, Nucl. Acids. Res. *9*, 1691—1706, 1981, modified in two ways. Firstly, *o*-chlorophenyl rather than *p*-chlorophenyl blocking groups were used for the phosphotriester grouping, and were incorporated in the mononucleotide and dinucleotide "building blocks". Figures 2 and 3 of the drawings show (a) dinucleotide and (b) mononucleotide "building blocks". DMT=4,4'-dimethoxytrityl and B=6-N-benzoyladenin-9-yl, 4-N-benzoylcytosin-1-yl, 2-N-isobutyrylguanin-9-yl or thymin-1-yl, depending on the nucleotide selected. Secondly, the "reaction cell" used for the successive addition of mono- or dinucleotide "building blocks" was miniaturised so that the coupling step with the condensing agent 1-(mesitylene-2-sulphonyl)-3-nitro-1,2,4-triazole (MSNT) was carried out in a volume of 0.5 ml pyridine containing 3.5 micromoles of polydimethylacrylamide resin, 17.5 micromoles of incoming dinucleotide (or 35 micromoles of mononucleotide) and 210 micromoles of MSNT.

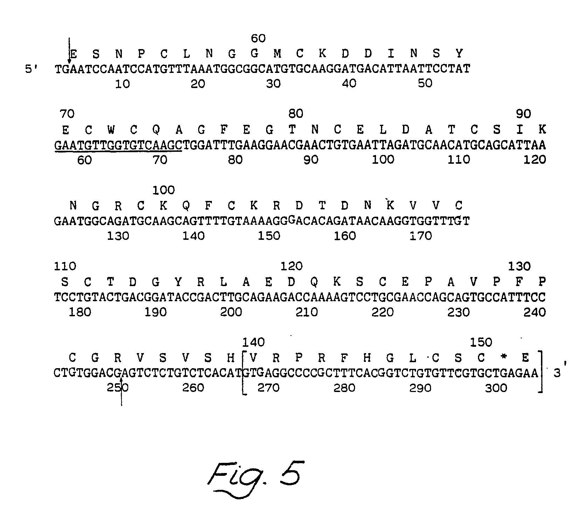

Figure 4 of the drawings is an elevational view of the microreaction cell 1 and stopper 2 used for oligonucleotide synthesis, drawn 70% of actual size. The device comprises a glass-to-PTFE tubing joint 3 at the inlet end of the stopper 2. The stopper has an internal conduit 4 which at its lower end passes into a hollow tapered ground glass male member 5 and thence into a sintered glass outlet 6 to the stopper. The cell 1 has a ground glass female member 7 complementary to the member 5 of the stopper, leading to reaction chamber 8, the lower end of which terminates in a sintered glass outlet 9. This communicates with glass tubing 10, and a 1.2 mm. "Interflow" tap 11. Further glass tubing 10, beyond the tap 11, leads to the outlet glass-to-PTFE tubing joint 12. Pairs of ears 13 on the stopper and cell enable them to be joined together by springs (not shown) in a liquid-tight manner.

After completion of the synthesis and deprotection, fractionation was carried out by high pressure liquid chromatography (Duckworth *et al.*, see above) and the peak tubes corresponding to the prdouct of correct chain length were located by labelling of fractions at their 5'-hydroxyl ends using [gamma-$^{32}$P]-ATP) and T4 polynuclotide kinase, followed by 20% 7M urea polyacrylamide gel electrophoresis. The position on the gel of the 17- and 14-oligonucleotides was determined by separately labelling, by the method described above, 17- and 14-nucleotide long "marker" oligonucleotides and subjecting these to the same gel electrophoresis.

D. Preparation of libraries of cDNA sequences for bovine mRNA

Two different approaches were used for the generation of cloned cDNA library:—

(i) *MboI* library

First strand cDNA was synthesised using the sucrose gradient-enriched poly(A)+bovine mRNA as template. The conditions used were as described by Huddleston & Brownlee, Nucl. Acids Res. *10*, 1029—1030, 1981, except that 2 micrograms of oligo N-1, 20—30 micrograms of the mRNA, 10 microcuries [alpha-$^{32}$P]-dATP (Amersham, 3000 Ci/mmole), and 50 U of reverse transcriptase were used in a 50 microlitre reaction. "dNTP" in Figure 1 denotes the mixture of 4 deoxynucleoside triphosphates required for synthesis. Oligo N-1 hybridises to the corresponding region on the mRNA (refer to Figure 1) and thereby acts as a primer for the initiation of transcription. It was used in order to achieve a further enrichment for factor IX mRNA. At the end of the cDNA synthesis reaction, the cDNA was extracted with phenol and desalted on a Sephadex-G100 column, before it was treated with alkali (0.1M NaOH, 1 mM EDTA) for 30 min at 60°C to remove the mRNA strand. Second strand DNA synthesis was then carried out exactly as published (Huddleston & Brownlee, Nucl. Acids Res. *10*, 1029—1038, 1981).

The double-stranded DNA was next cleaved with the restriction enzyme *MboI* and ligated to the plasmid vector pBR322 which had been cut with *Bam*HI and treated with calf intestinal alkaline phosphatase to minimise vector self-religation. Phosphatase treatment was carried out by incubating 5 micrograms of *Bam*HI-cut pBR 322 plasmid with 0.5 microgram calf intestinal phosphatase (Boehringer; in 10 mM Tris—HCl buffer, pH 8.0) in a volume of 50 microlitres at 37°C for 10 minutes, see Huddleston & Brownlee *supra*.

The ligated DNA was used to transform *E. coli* strain MC 1061. For transformation *E. coli* MC 1061 was grown to early exponential phase as indicated by an absorbancy of 0.2 at 600 nm and made "competent" by treating the pelleted bacterial cells first with one half volume, followed by repelleting, and then with 1/50 volume of the original growth medium of 100 mM $CaCl_2$ 15% v/v glycerol and 10 mM PIPES-NaOH, pH 6.6 at 0°C. Cells were immediately frozen in a dry ice/ethanol bath to −70°C. For transformation, 200 microlitre aliquots were mixed with 10 microlitres of the recombinant DNA and incubated at 0°C for 10 minutes followed by 37°C for 5 minutes. 200 microlitres of L-broth (bactotryptone 10 g., yeast extract 5 g., sodium chloride 10 g., made up to 1 litre with deionised water) were then added and incubation continued for a further 30 minutes at 37°C. The solution was then plated on the appropriate antibiotic agar (see below). A library of about 7,000 ampicillin-resistant colonies was thus obtained. They were ampicillin-resistant because they contained the beta-lactamase gene of pBR 322. Of these, approx. 85% were found to be tetracycline-sensitive.

(ii) dC/dG tailed library

In the preparation of this library, first strand cDNA was synthesised as described for the above library except that oligo $dT_{(12-18)}$ was used as primer to initiate cDNA synthesis. Following this, the cDNA was tailed with dCTP using terminal transferase and back-copied with the aid of oligo $dG_{(12-18)}$ primer and reverse transcriptase to give double stranded DNA, exactly according to the method of Land *et al.,* Nucl. Acids Res. *9*, 2251—2266, 1981. After a further tailing with dCTP, this material was annealed by hybridisation to a dGTP-tailed pBR322 plasmid at the *Pst*I site. The hybrid DNA was used to transform *E. coli* strain MC 1061. A library of approximately 10,000 tetracycline-resistant colonies was obtained. Of these, approximately 80% were found to be sensitive to ampicillin, due to insertion of DNA into the ampicillin-resistant gene at the *Pst*I site.

E. Isolation of specific bovine factor IX clones

(i) From *MboI* library

The library of colonies, in an unordered fashion, was transferred onto 13 Whatman 541 filter papers and amplified with chloramphenicol, to increase the number of copies of the plasmid in the colonies, as described by Gergen *et al.* Nucl. Acids Res. *1*, 2115—2136 (1979). The filters were pre-hybridised at 65°C for 4 h in 6×NET (1×NET=0.15 m NaCl, 1 mM EDTA, 15 mM Tris-HCl, pH 7.5), 5×Denhardt's, 0.5% NP40 nonionic surfactant, and 1 microgram/ml yeast RNA as described by Wallace *et al.,* Nucl. Acids. Res. *9*, 879—894 (1981). Hybridisation was carried out at 47°C for 20 h in the same solution containing $3×10^5$ cpm (0.7 nanogram/ml) of labelled oligo N-2 probe. Labelling was done by phosphorylation of the oligonucleotides at the 5' hydroxyl end using [gamma-$^{32}$P]-ATP and T4 phosphokinase (Huddleston & Brownlee, Nucl. Acids. Res. *10*, 1029—1038, 1981). At the end of the hybridisation, filters were washed successively at 0—4°C (2 h), 25°C (10 min), 37°C (10 min) and 47°C (10 min). After radioautography of the filters from this screening, one colony showed a positive signal above background. This colony was designated BIX-1 clone.

(ii) from dC/dG-tailed library

Screening of this library, in an ordered array fashion, using oligo N-2 probe as described above has resulted in the identification of a positive clone. This was designated BIX-2 clone.

F. Sequence characterisation of bovine factor IX cDNA clones

Characterisation of BIX-1 clone by restriction endonuclease cleaveage indicated that it contained a DNA insert of about 430 base-pairs (data omitted, for brevity). Figure 5 shows part of the nucleotide sequence of the coding strand, determined by the Maxam-Gilbert method, extending over 304 nucleotides and provides direct evidence that it has the identity of a bovine factor IX sequence. Thus, nearly all of this 304 nucleotide sequence (corresponding to amino acid residues 52—139) agrees with the nucleotide sequence predicated from the known bovine factor IX amino acid sequence data (Katamaya *et al.,* Proc. Natl. Acad. Sci. *76*, 4990—4994, 1979). Over this region, there are no discrepancies between BIX-1 and these published data for factor IX, except at nucleotides 38—40 where the amino acid coded for is Asp instead of Thr. This amino acid change was similarly observed in a second, independent cDNA clone (BIX-2; see below). The remainder of the 304-nucleotide sequence, i.e. that shown in brackets in Figure 5, does not agree with the published bovine factor IX amino acid data of Katayama.

In Figure 5, the underlined portion denotes the sequence corresponding to the oligo N-2 probe sequence, the asterisk denotes a nonsense codon, the brackets enclose a sequence which does not correspond to Katayama's amino acid data and the arrows indicate *Hinf*I restriction sites. The Katayama numbering system for amino acids is shown and this sequence is in the opposite orientation to the direction of transcription of the tetracycline-resistant gene of the plasmid.

By similar methods, BIX-2 clone was found to have a DNA insert of 102 nucleotides and this spans the nucleotide positions 7-108 as shown in Figure 5. The nucleotide sequences for BIX-1 and BIX-2 clones over this region (nucleotide 7-108) were identical.

G. Isolation of human factor IX gene
(i) Initial clone—lambda HIX-1

A library of cloned human genomic DNA, namely a *Hae*III/*Alu*I lambda phage Charon 4A library prepared by Lawn *et al.,* Cell, *15*, 1157—1174, 1978, was used. $10^6$ phage recombinants from this library were screened using the *in situ* plaque hybridisation procedure as described by T. Maniatis *et al.,* Cell, *15*, 687, 1978. Pre-hybridisation and hybridisation were carried out at 42°C in 50% formamide. After hybridisation, filters were washed at room temperature with 2×SSC (1×SSC=0.15 mM NaCl, 15 mM sodium citrate, at pH 7.2) and 0.1% SDS, then at 65°C with 1×SSC and 0.1% SDS.

Two DNA molecules, being restriction fragments from the factor IX cDNA cloned in BIX-1, were radiolabelled and used as probes in the hybridisation. The first fragment corresponds to nucleotide numbers −8 to 317 on the numbering system of Figure 5, and was isolated by *Sau*3AI digestion of BIX-1 plasmid DNA. The isolated DNA was labelled to high specific activity by incorporation of [alpha-$^{32}$P]-dATP using a nick translation (Rigby *et al.,* J. Mol. Biol. *113*, 237—251, 1977, modified, *vide infra*). Using this probe, 10 clones were isolated. These were plaque-purified and re-hybridised with a 247-nucleotide fragment from BIX-1 clone. This fragment, derived from nucleotides 3-249 can be seen from Figure 5. It contains only sequences in agreement with the Katayama bovine factor IX amino acid sequence and was isolated by *Hinf*I digestion of BIX-1 plasmid DNA. Only a single clone gave a positive hybridisation signal with this 247-nucleotide probe. This clone was further plaque-purified and the resulting clone was designated "lambda HIX-1".

(ii) Subsequent genomic clones

A sub-clone, pATIXcVII, of recombinant human factor IX cDNA from human liver mRNA, and prepared as described in Section L below, was linearised by digestion with *Hind*III and *Bam*HI. The resulting 2 kb cDNA molecule was purified by 1% agarose gel electrophoresis. After electroelution, about 100 ng of this cDNA was nick-translated with [alpha$^{32}$p] dATP (see above) and used as a hybridisation probe to screen the *Hae*III/*Alu*I lambda phage Charon 4A human genomic DNA library for further genomic clones, using standard stringent hybridisation conditions. Two further human factor IX genomic clones, designated lambda HIX-2 and lambda HIX-3, were thus obtained.

H. Characterisation of human factor IX genomic clones
(i) Restriction map

The initial lambda HIX-1 clone was characterised by cleavage with various single and double digests with different restriction endonucleases and Southern blotting of fragments using the bovine factor IX cDNA probe (results omitted for brevity). The subsequently isolated lambda HIX-2 and 3 clones were characterised in the same way except that the human cDNA probe, pATIXcVII (see Section L below) was used for the Southern blots. From these results it emerged that the sequences in the factor IX genome corresponding to lambda HIX-2 and 3 overlapped with lambda HIX-1 as shown in Figure 6(e). In Sectin (d) of this Figure 6 are summarised the results of the analysis using the restriction enzymes *Eco*RI (E), *Hind*III (H), *Bgl*II(B), *Bam*HI (Ba) and *Pvu*II (P), and this serves as a restriction enzyme map.

(ii) Sequencing

Numerous sub-clones were isolated from a knowledge of the restriction enzyme map as described in Section J(ii) below, the majority in a vector pAT153/Pvull/8. Examples of these sub-clones are shown in Figure 6(c) and a number were used and were of a convenient length for sequence analysis by the Maxam-Gilbert method (Maxam & Gilbert, Proc. Natl. Acad. Sci. USA *74*, 56—564, 1980).

Initially, sequencing was done on part of a 1.4 kb *Eco*RI restriction fragment from the sub-clone pHIX-17, see below and J(i). A 403-nucleotide (base-pair) length was sequenced, of which a 129-nucleotide length was identified as lying within an exon region. This is the 129-nucleotide sequence used above to define the factor IX DNA.

Subsequently, a region of 11873 bases was sequenced in the central portion of the gene [see Figure 6(b)]. Figure 7 shows the sequence of one strand of the DNA. The nucleotides are arbitrarily numbered from 1 to 11873 in the 5' to 3' direction. The original 403-nucleotide sequence runs from Figure 7 nucleotides Nos. 4372 to 4774 and is indicated by 0—0'. The 129-nucleotide sequence lying within the 403 one, runs from Figure 7 nucleotides Nos. 4442 to 4570 and is indicated by J—J'. This corresponds exactly to the "w" exon.

In detail, the sequence of nucleotides Nos. 1—7830 contains two short exons (nucleotides 4442—4570 and 7140—7342 respectively) marked w and x in Figures 6(a) and 9, J—J' and J'—J'' in Figures 7 and 9.

These code for amino acids 85—127, and 128—195 respectively of the amino acid sequence predicted from the human factor IX cNDA clone (Figure 9). There are no differences in amino acid sequences predicted from the genomic and cDNA clones of the invention in these two exon regions. The sequence of the gene between residues 7831—11873 is less complete, containing several gaps, but is still a useful characterisation of the gene as it contains two "*Alu*l repeat" sequences, nucleotides 7960—8155 and 9671—9938. *Alu*l sequences are found in many genes. The repetition is not exact but there is a typical degree of homology between them. This further characterisation provides a useful cross-check on the accuracy of the restriction enzyme map. This emerges more clearly from the restriction enzyme chart of Figure 8.

Figure 8 is a chart produced by a computer analysis of the sequence data of the 11873 nucleotide long sequence of Figure 7. Column 1 of Figure 8 gives the arbitrary nucleotide number allotted to the nucleotide of Figure 7. Column 2 apportions the nucleotide number as a fraction of the whole sequence. Column 3 shows the restriction enzymes which will cut the DNA within various short sequences of nucleotides shown in Column 4. The short sequences of Column 4 begin with the nucleotide numbered in Column 1. With the aid of this chart the positions of the restriction sites shown in Figure 6(d) and some of the sequences shown in Figure 6(c) can be determined very accurately. For example sequences II—IV are produced by restriction at the following sites (denoted by the first nucleotide number at the 5' end of each site).

| | |
|---|---|
| II | 3624—4769 |
| III | 6380—7378 |
| IV | 10589—11868 |

Particularly important sites are arrowed in Figure 8. Some of the relevant nucleotide numbers are shown in Figure 6(c), the number given being that of the nucleotide at the 5' end of each site.

Further sequence analysis of the sub-clones V, VI, VII and VIII shown in Figure 6(c) indicates that the factor IX gene is divided into at least 7 exon regions separated by at least 6 introns. The positions of the exons are shown in Figure 6(a) by the solid blocks labelled t, u, v, w, x, y and z. The 'z" exon is much the longest and its 3'-end coincides with the 3'-end of the MRNA. The location of these exons relative to the cDNA sequence is discussed below (section L) and it is clear that the "t" exon shown in Figure 6(a) is not a marker for the 5'-end of the gene, as its sequence fails to match that of the extreme 5'-end of the cDNA clone (see below). This suggests that the factor IX gene will be longer at its 5'-end than the 27 kb region shown in Figure 6, and will contain at least one further exon.

Additionally, pHIX-17 DNA was digested with *Eco*RI. The digested material was resolved on 0.8% agarose gel and a 1.4 kb fragment was isolated in solution by electroelution. It can be stored in the usual manner. This 1.4 kb long molecule was used for the initial sequencing. Only about 1.0 kb is inserted DNA, the remaining 0.4 kb being of pBR322. A 403 nucleotide length of the inserted DNA was sequenced and is identified as 0—0' in Figure 7. The same 1.4 kb fragment was also labelled and used as a probe in Section M.

I. Construction of a vector pAT153/PvuII/8

A derivative of the plasmid pAT153 (Twig & Sherratt, Nature *283*, 216—218, 1980) was prepared for subcloning of *Pvu*II fragments of factor IX genomic clones, and for ease of characterisation of the resultant subclones. Two partially complementary synthetic deoxyoligonucleotides, oligo N3, and, oligo N4, were synthesised by the solid phase phosphotriester method described in Section C above. Each has "overhanging" *Bam*HI and *Hind*III recognition sequences and an internal *Pvu*II recognition sequence. Figure 10 shows the structures of oligo N3 and oligo N4. *Bam*HI and *Hind*III cleave ds DNA to leave sticky or "overhanging" ends. For example *Hind*III cleaves

—AAGCTT
—TTCGAA

between the adenine-carrying nucleotides of each strand leaving the sticky-ended complementary strands:—

—A
—TTCGA

which are present in the oligo N3/N4 combination.

pAT153 was digested with *Hind*III and *Bam*HI and the 3393 nucleotide long linear fragment was separated from the 346 nucleotide shorter fragment by 0.7% agarose gel electrophoresis, followed by electroelution of the appropriate bands visualised by ethidium bromide fluorescence under UV light. After treatment with calf intestinal phosphatase, as described in Section D(i), the *Bam*HI-*Hind*III 3393-long fragment was ligated to an equimolar mixture of oligo N3 and oligo N4 which themselves had been pretreated, as a mixture, with p4 polynucleotide kinase and ATP, to phosporylate their respective 5'-terminal OH groups. After transforming competent MC 1061 cells (see above) and plating on L-broth plates containing 20 micrograms/ml final concentration of ampicillin, 11 colonies were selected for further analysis. 1 ml plasmid preparation, see Holmes and Quigley, Analytical Biochem. *114*, 193—197 (1981), was isolated from the 11 colonies. The plasmid DNA was then analysed for its ability to be linearised by the restriction enzymes *Bam*HI, *Hind*III and *Pvu*II. Four clones were positive in this assay and one, labelled pAT153/PvuII/8, was selected for sequence analysis by the Maxam-Gilbert method across the newly constructed section of the plasmid. This part of the sequence is shown in Figure 11 along the unique restriction sites. The novel part of the plasmid sequence is underlined: the remainder is present in the parent plasmid pAT153. The vector allows blunt-end cloning (after treatment with phosphatase) into the inserted *Pvu*II site. The cloned DNA can be excised, assuming that it lacks appropriate internal restriction sites, with *Bam*HI/*Hind*III, *Bam*HI/*Cla*I or *Bam*Hi/*Eco*RI double digests. The sites adjacent to the *Pvu*II site are also convenient for end labelling with $^{32}$P for characterization of the ends of cloned DNA by the Maxam-Gilbert sequencing method.

## J. Sub-cloning of human factor IX gene

The following subcloning experiments were carried out as a first step towards sequencing of the factor IX gene, and to facilitate the isolation of a small DNA fragment to be used as a probe for the analysis of genomic DNA from haemophilia B patients (see sections M).

### (i) Sub-cloning into pBR322 plasmid

An approximately 11 kilobase *Bg*/II fragment (see Figure 6) within the factor IX DNA insert in lambda HIX-1 clone was inserted into the *Bam*HI site of pBR322 Transformation was carried out in the *E. coli* strain, HB 101. The resulting "sub-clone" was designated pHIX-17 (Figure 12).

### (ii) Sub-cloning into pAT/153/PvuII/8

(a) Plasmid DNA from pHIX-17 was prepared and cleaved with *Pvu*II. Five discrete fragments, all derived from the DNA insert of pHIX-17, were isolated. The sizes of these fragments were approximately 2.3, 1.3, 1.2, 1.1 and 1.0 kilobases. These fragments were blunt-end ligated into the *Pvu*II site of the pAT153/PvuII/8 vector and transformed into *E. coli* HB 101. Five clones of recombinant DNA which carried the 2.3, 1.3, 1.2, 1.1 and 1.0 kb fragments were obtained and these were designated pATIXPvu-1, 2, 3, 4 and 5 respectively. Factor IX DNA from pATIXPvu-2 is abbreviated as IV and pATIXPvu-5 as III in Figure 6(c).

(b) Phage DNA from the lambda HIX-1 genomic clone was digested with *Eco*RI. Three different fragments (approximately 5, 2.3, 0.96, kb; see Figure 6), all derived from the insert into the phage, were isolated and inserted in pAT153/PvuII/8 vector at the *Eco*RI site and cloned in *E. coli* HB 101 to form sub-clones. The three resulting clones for each of these fragments were designated pATIXEco-1, 2 and 4 respectively which are shown in the restriction map of Figure 6(d). pATIXEco-1 was further digested with both *Eco*RI and *Bg*/II, and the "overhanging ends" of the restriction sites filled in with deoxynucleoside triphosphates using the Klenow fragment of DNA polymerase I. After isolation of the resulting 1.1 kb fragment by agarose gel electrophoresis and electroelution, it was blunt-end ligated using T4 DNA ligase into the *Pvu*II site of pAT153/PvuII and allowed to transform *E. coli* MC 1061. The resultant sub-clone was designated pATIXBE and the factor IX DNA sequence thereof is abbreviated as II in Figure 6(c).

(c) Phage DNA from lambda HIX-2 was digested with *Hind*III and *Eco*RI giving a 1.8 kb and a 2.6 kb fragment amongst others. These fragments were eluted separately, filled in as described in (b) above, cloned as above into the *Pvu*II site of pAT153/PvuII/8 and allowed to transform *E. coli* MC 1061. The resultant clones were designated pATIXHE-1, and the factor IX DNA sequence thereof is abbreviated as V in Figure 6(c), and pATIXEco-6 and the factor IX DNA sequence thereof is abbreviated as VI in Figure 6(c).

(d) Phage DNA from lambda HIX-3 was digested with *Eco*RI and *Hind*III and the fragments of 2.3 kb and 2.7 kb were sub-cloned exactly as described in (c) above. The resultant clones were designated pATIXEH-1, abbreviation VII in Figure 6(c), and pATIXHE-2, abbreviation VIII in Figure 6(c).

## K. Preparation of a library of cDNA clones from human liver mRNA

Messenger RNA was extracted from a human liver and a 20—22 Svedberg unit enriched fraction of mRNA prepared exactly as described for bovine mRNA in Section B above, except that a "translation assay' was not used. The first steps in the construction of the double-stranded DNA were carried out using the 'Stanford protocol' kindly supplied from Professor P Berg's department at Stanford University, USA. This itself is a modification of Wickens, Buell & Schimke (J. Biol. Chem. *253*, 2483—2495, 1978) and some further modifications, incorporated in the description given below were made in the present work.

For the first strand cDNA synthesis 6 micrograms of poly(A)+20—22S human mRNA was incubated with 5 microlitres of 10× buffer (0.5 M Tris-chloride, pH 8.5 at room temperature, 0.4 M KCl, 0.08M MgCl$_2$ and 4 mM dithiothreitol), 20 microlitres of a 2.5 mM mixture of each of the four deoxynucleoside triphosphates, 0.5 microlitres of oligo dT$_{(12-18)}$, 1 microlitre (containing 0.5 microcurie) of [alpha-$^{32}$P]-dATP, 2 microlitres of reverse transcriptase (14 units per microlitre) and the volume made up of 50 microlitres with deionized water. After incubation for 1 hour at 42°C, the solution was boiled for 1½ minutes and then rapidly cooled on ice. The second strand synthesis was carried out by adding directly to the above solution 20 microlitres of 5× second strand buffer (250 mM Hepes/KOH pH 6.9, 250 mM KCl, 50 mM MgCl$_2$), 4 microlitres of a 2.5 mM mixture of each of the four deoxynucleoside triphosphates, 10 microlitres of *E. coli* DNA polymerase I (6 units per microlitre) and making the volume of the solution up to 100 microlitres with deionized water. After incubation for 5 hours at 15°C, $S_1$ nuclease digestion was carried out by the addition of 400 microlitres of $S_1$ nuclease buffer (0.03 M sodium acetate pH 4.4, 0.25 M NaCl, 1 mM $ZnSO_4$) and 1 microlitre of $S_1$ nuclease (at 500 units per microlitre). After incubating for 30 minutes at 37°C, 10 microlitres of 0.5 m EDTA (pH 8.0) was added. Double stranded DNA was deproteinised by shaking with an equal volume of a phenol:chloroform (1:1) mixture, followed by ether extraction of the aqueous phase and precipitation of ds DNA by addition of 2 volumes of ethanol. After 16 hours at −20°C, ds DNA was recovered by centrifugation. DNA polymerase I "fill in" of $S_1$ ends was carried out by a further incubation of the sample dissolved in 250 microlitres of 50 mM tris-chloride, pH 7.5, 10 mM $MgCl_2$, 5 mM dithiothreitol and containing 0.02 mM dNTP and 6 units of DNA polymerase I. After incubating for 10 minutes at room temperature, 5 microlitres of EDTA (0.1 M at pH 7.4) and 3 microlitres of 5% sodium dodecyl sulphate (SDS) were added.

The following part of the protocol differs from the 'Stanford protocol'. The sample was fractionated on a "mini"-Sephacryl S400-column run in a disposable 1 ml pipette in 0.2 M NaCl, 10 mM Tris-chloride, pH 7.5 and 1 mM EDTA. The first 70% of the "breakthrough" peak of radioactivity was pooled (0.4 ml) and deproteinised by shaking with an equal volume of n-butanol:chloroform (1:4). To the aqueous phase was added 1 microgram of yeast RNA (BDH) as carrier followed by 2 volumes of ethanol. After 16 hours at −20°C double stranded DNA was recovered by centrifugation for blunt-end ligation into calf intestinal phosphatase-treated, PvuII-cut pAT153/PvuII/8, using T4 DNA ligase (see I and J(ii) above). After performing a trial experiment, it was found that when the bulk of the sample was incubated with 200 nanograms of vector DNA in a suitable buffer (1 mM ATP, 50 mM Tris-chloride, pH 7.4, 10 mM $MgCl_2$ and 12 mM dithiothreitol) and using 10 microlitres of T4 DNA ligase in a total volume of 0.2 ml, then on subsequent transformation of competent E. coli MC 1061 cells a total of 58,000 ampicillin-resistant colonies were obtained. Up to 20% of these were estimated to derive from "background" non-recombinants derived by religation of the vector itself. This 20—22S cDNA library was amplified by growing the E. coli for a further 6 hours at 37°C. 1 ml aliquots of this amplified library were stored at −20°C in L broth containing 15% glycerol, before screening for factor IX cDNA clones.

L. Isolation and sequence analysis of human factor IX cDNA clones

6000 colonies of the amplified 20—22S human cDNA library were plated on each of ten 15 cm agar plates and after growing overnight were blotted into Whatman 541 filter paper. After preparing filters for hybridisation as described in section E(i) above, the immobilised colonies were probed with a 1.1 kb molecule of [alpha-$^{32}$P]-nick translated human factor IX genomic DNA isolated from the pATIXBE subclone (Section J, above). This linear 1.1 kb section of factor IX genomic cDNA was isolated from pATIXBE by cleavage with the restriction enzymes BamHI and HindIII, followed by separation of the 1.1 kb section from the vector by 1.5% agarose gel electrophoresis. After electroelution, nick-translation was carried out as before and the material used in a hybridisation reaction for 16 hours at 65°C in 3× SSC, 10× Denhardts solution, 0.1% SDS and 50 micrograms/ml sonicated denatured E. coli DNA and 100 micrograms/ml of sonicated denatured herring sperm DNA. After hybridisation filters were charged at 65°C successively in 3× SSC, 0.1% SDS (2 changes, half an hour each) and 2× SSC, 0.1% SDS (2 changes, half an hour each). After radioautography, 7 clones were selected as positive, but on dilution followed by re-screening by hybridisation as above, only 5 proved to be positive. Plasmid DNA was isolated from each of these 5 clones and one, designated pATIXcVII, was selected for sequence analysis as it appeared to be the longest of the 5 clones as judged by its electrophoretic mobility on 1% agarose gel electrophoresis. A second shorter clone, designated pATIXcVII was also selected for partial sequence analysis.

Sequencing was carried out by the Maxam-Gilbert method and a 2778 nucleotide long section of sequence is shown in Figure 9. Nucleotides 115—2002 were derived by sequencing clone pATIXcVII. (The actual extent of this clone is greater as it extends in a 5′ direction to nucleotide 17. The sequence between 17 and 111 is inverted with respect to the remainder of the sequence presumably due to a cloning artefact). Nucleotides 1—130 were derived from clone pATIXcVI which extends from nucleotides 1—1548 of Figure 9. The sequence from Nos. 2002—2778 was derived by isolating 4 additional clones designated pATIX108.1, pATIX108.2, pATIX108.3 and pATIXDB. The first 3 were derived from a mini-library (designated GGB108) of cDNA clones constructed exactly as described in section K above except that sucrose density gradient centrifugation was used instead of chromatography on "Sephacryl" S-400 to fractionate the double-stranded DNA according to size. A fraction of m.w. from 1 kb—5 kb was selected and an amplified library of 10,000 independent clones containing approximately 20% background non-recombinant clones was obtained. Clone pATIXDB derived from another cDNA library (designated DB1) constructed as described in section K except that total poly A+ human liver mRNA was used as the starting material and sucrose density gradient centrifugation was used to fractionate the DNA according to size as in the construction of the mini-library GGB108. The complexity of this library was 95,000 with an estimated background of non-recombinants of 50%. Clones pATIX108.1 and pATIX108.2 were selected from a group of 30 hybridization-positive clones isolated by Grunstein-Hogness screen of the mini library GGB108 using a $^{32}$P-nick translated probe derived from a Sau3AI restriction enzyme fragment, itself derived from nucleotides 1796—2002 of clone pATIXcVII. From pATIX108.1 the sequence of nucleotides 2009—2756 was determined (Figure 9). Following this the sequence of a part of pATIX108.2, specifically nucleotides 1950—2086, provided the overlap with pATIXcVII. The remaining 28 hybridization positive clones were screened by carrying out a triple enzymatic digestion with the restriction enzymes *Eco*RI, *Bam*HI and *Hind*III and screening the product of the digest for an *Eco*RI restriction fragment extending in the 3' direction from the cut at position 2480. By this approach, clone pATIX108.3 was selected and sequenced from nucleotides 2642—2778. This clone was followed by three A nucleotides, which sequence was confirmed as a vestigial marker for the poly A tail, by the subsequent isolation of clone pATIXDB by a similar method. pATIXDB was sequenced from Nos. 2760—2778 and ended in 42 A nucleotides, thus marking the 3' end of the mRNA.

Figure 9 shows that the predicted amino acid sequence codes for a protein of 456 amino acids, but included in this are 41 residues of precursor amino acid sequence preceding the N-terminal tyrosine residue ( Ẏ) of the definitive length factor IX protein. The precursor section of the protein shows a basic amino acid domain (amino acids −1 to −4) as well as the more usual hydrophobic signal peptide domain (amino acids −21 to −36).

The definitive factor IX protein consists of 415 amino acids with 12 potential gamma-carboxyglutamic acid residues at amino acids 7, 8, 15, 17, 20, 21, 26, 27, 30, 33, 36 and 40. Two potential carbohydrate attachment sites occur at amino acid residues 157 and 167. The activation peptide encompasses residues 146—180, which are cut out in the activation of Factor IX (see Background of Invention) by the peptide cleavage of an R—A and R—V bond. This leaves a light chain spanning residues 1—145 and a heavy chain spanning residues 181—415.

The exact location of the boundaries between exons (see Section H, above) and how they are joined in the mRNA is marked in Figure 9. The exons are marked t, u, v, w, x, y, z. It can be seen that there is a rough agreement between the exon domains and the protein regions. For example, the exon for the signal peptide is distinct from that of the GLA region. Also that of the activation peptide is separated from the serine protease domain.

The 3' non-coding region of the mRNA is extensive, consisting of 1390 residues (including the UAAUGA double terminator 1389—1394 but excluding the poly A tail).

The factor IX cDNA is cleavable by the restriction enzyme *Hae*III to give a fragment from nucleotides 133—1440, i.e. a 1307 nucleotide long region of DNA entirely encompassing the definitive factor IX protein sequence. The nucleotide sequence recognised by *Hae*III is GGCC. This fragment should be a suitable starting material for the expression of factor IX protein from suitable promoters in bacterial, yeast or mammalian cells. Another suitable fragment could be derived using the unique *Stu*I site at residue 41 (corresponding to an early part of the hydrophobic signal peptide region) and linking it to a suitable promoter. The nucleotide sequence recognised by *Stu*I is AGGCCT.

M. Southern Analysis of normal and patient Christmas disease DNA
(i) Normal

The standard (Southern) blotting procedure, Southern, J. Mol. Biol. *98*, 503—517, 1975) was used. In a typical experiment, 10—20 micrograms of human genomic DNA (prepared from uncultured blood cells or cultured lymphocytic cells) were digested with one of a number of restriction endonucleases and loaded onto a single gel slot. Following electrophoresis on 0.8% agarose gel and transfer onto nitrocellulose it was hybridised with a probe of $^{32}$P-labelled probe II or of 1.4 kb *Eco*RI fragment (see Section H). Labelling of the probe was carried out by nick translation using the method of Rigby *et al., supra*, modified as follows. About 100 nanograms of the probe was mixed with 40 microcuries of [alpha $^{32}$P] dATP (activity about 3,000 Curies/mMole, obtained from Amersham International PLC) in 0.05M Tris-HCl, pH 7.5, 0.01M MgCl$_2$, 0.001M dithiothreitol and dCTP, dGTP, dTTP each at a final concentration of 20 micromolar in a volume of 29 microlitres. To this was added 1 microlitre of "solution X" made up of a mixture of 6 units of DNA polymerase I (*E. coli*), 0.6 nanograms of pancreatic DNase I (Wothington), 1 microgram of crystalline BSA in 10 microlitres of 50% v/v glycerol containing 0.05M Tris-HCl, pH 7.5, 0.01M MgCl$_2$ and 0.001M dithiothreitol. The mixture was incubated for 2 hours at 15°C, after which high molecular weight DNA was purified by chromatography on G-100 "Sephadex". Figure 13 shows the major bands obtained with DNA from normal individuals probed with either probe II (Figure 6) or labelled 1.4 kb *Eco*RI fragment. With each of the 4 enzymes used, *Eco*RI, *Hind*III, *Bg*/II and *Bc*/I, a single major band of about 4.8, 5.2, 11 and 1.7 kb was obtained.

The fact that these restriction fragments had the same length as those observed in the restriction map of clone lambda HIX-1 confirmed that the conditions of Southern blotting were precise enough to detect the factor IX gene in total DNA preparations. This provides the basis for analysis of DNA from the blood of patients with Christmas disease.

(ii) Christmas patients with gene deletions

The value of the probes of the invention for the assay of alterations of genes of some patients suffering from Christmas disease has been demonstrated as follows. Two patients with severe Christmas disease, who also developed antibodies to factor IX, were selected for study. The DNA from 50 ml of blood was digested separately with *Eco*RI, *Hind*III, *Bg*/II and *Bc*/I and Southern blots prepared for probing with $^{32}$P-nick translated probe II (Figure 6). No specific bands were observed with either patient under conditions where a control digest gave the pattern shown in Figure 13. Similarly no bands were observed in the patients when probe I, III or IV (Figure 6) was substituted for probe II. In order to control for possible mischance of some experimental artefact giving the observed 'negative' signal, a factor IX gene probe (this time pATIXc-

# EP 0 107 278 B1

VII—the cDNA probe) was mixed with an irrelevant autosomal gene probe which was specific for the human AI apolipoprotein (Shoulders and Baralle, Nucl. Acids. Res. *10*, 4873—4882, 1982). This experiment showed that patient 1 had the normal AI apolipoprotein gene, characterised by a 12 kb band in an *Eco*RI digest, and confirmed that he lacked the 5.5 kb band observed with pATIXcVII and characteristic of the normal factor IX gene. It was concluded that both patients have a seqeunce of at least 18 kb deleted from their factor IX gene. Two other patients, designated patients 3 and 4, who had also developed antibodies to factor IX gave bands in the normal or abnormal positions on Southern blots with some factor IX gene probes of the invention, but not with others. This suggested that these patients had less extensive deletions of the gene, possibly about 9 kb in length.

These results suggest that diagnosis of haemophiliacs and the heterozygous (carrier) females would be possible in families and this is now under examination. The altered pattern seen in the patient's DNA, whether absence of a band or the presence of a band in an abnormal position, serves as a "disease marker", which can be used to assess for its presence or absence in a suspected carrier. This same test can be applied to antenatal diagnosis, if DNA from foetal cells are available from an aminocentesis. "Genetic diagnosis" should considerably improve existing methods of antenatal diagnosis based on the assay of foetal factor IX protein levels, with the added advantage that the test can be carried out earlier in pregnancy. Genetic methods using natural polymorphisms within the factor IX gene as allelic markers should also make 100% carrier detection a reality, thereby improving the existing somewhat unsatisfactory methods where probability values are offered to patients.

Deposits have been made at the National Collection of Industrial Bacteria, Torry Research Station, P. O. Box 31, 135 Abbey Road, Aberdeen AB9 8DG Scotland, as follows:

(1) the phage present in the clone hereinbefore designated lambda HIX-1 deposited as a phage, under Accession No. 11749 on 30 July 1982;

(2) and *E. coli* strain containing the modified pAT153 plasmid (hereinbefore designated pAT153/Pvu II/8) under Accession No. 11747 on 19 July 1982;

(3) The *E. coli* clone hereinbefore designated BIX-1 containing a recombinant bovine factor IX DNA, under Accession No. 11748 on 19 July 1982; and

(4) *E. coli* K-12 strain 803 mentioned above, which is a suitable host for the lambda HIX-1b phage, under Accession No. 11752. All deposits were made under the provisions of the Budapest Treaty on the International Recognition of the Deposit of Micro-organisms for the Purposes of Patient Procedure.


## Claims for the Contracting States: BE CH DE FR GB IT LI LU NL SE

1. An artificial DNA molecule produced by recombinant DNA technology and encoding a precursor of human factor IX polypeptide which is convertible *in vivo* to the human factor IX polypeptide by the action of a vitamin K-dependent enzyme, or useful as a hybridisation probe for the diagnosis of Christmas disease, said DNA comprising substantially the following 129-nucleotide sequence (read in rows of 30 across the page):—

<pre>
(5')  ATGTAACATG    TAACATTAAG    AATGGCAGAT
      GCGAGCAGTT    TTGTAAAAAT    AGTGCTGATA
      ACAAGGTGGT    TTGCTCCTGT    ACTGAGGGAT
      ATCGACTTGC    AGAAAACCAG    AAGTCCTGTG
      AACCAGCAG (3')
</pre>

and/or its complement.

2. A DNA molecule according to Claim 1 which is complementary to human factor IX messenger RNA, said mRNA being transcribable from the human factor IX genome.

3. Recombinant DNA which comprises a cloning vehicle DNA sequence and a sequence foreign to the cloning vehicle, the foreign sequence being as claimed in Claim 1 or 2.

4. A host cell transformed with recombinant DNA claimed in Claim 3.

5. A host cell according to Claim 4 in the form of a bacterium or yeast.


## Claims for the Contracting State: AT

1. A recombinant DNA process in which foreign DNA is inserted in a cloning vehicle, the cloning vehicle is replicated in a host cell, recombinant DNA is recovered from the host and optionally the foreign DNA is excised from the recombinant DNA containing it, characterised in that the foreign DNA encodes a precursor of human factor IX polypeptide which is convertible *in vivo* to the human factor IX polypeptide by the action of a vitamin K-dependent enzyme, or is useful as a hybridisation probe for the diagnosis of Christmas disease, said DNA, being further characterised in that the foreign DNA comprises substantially the following 129-nucleotide sequence (read in rows of 30 across the page):—

14

(5') ATGTAACATG    TAACATTAAG    AATGGCAGAT
GCGAGCAGTT    TTGTAAAAAT    AGTGCTGATA
ACAAGGTGGT    TTGCTCCTGT    ACTGAGGGAT
ATCGACTTGC    AGAAAACCAG    AAGTCCTGTG
AACCAGCAG (3')

and/or its complement.

2. A process according to Claim 1 characterised in that the foreign DNA is complementary to human factor IX messenger RNA, said mRNA being transcribable from the human factor IX genome.

3. A process according to any preceding claim characterised in that the host cell is a bacterium or yeast.

## Patentansprüche für die Vertragsstaaten: BE CH DE FR IT LI LU NL SE

1. Ein künstliches DNA-Molekül, hergestellt durch rekombinante DNA-Technologie, das eine Vorläuferform des humanen Faktor IX-Polypeptids kodiert, die in vivo durch Einwirkung eines Vitamin K-abhängigen Enzyms in das humane Faktor IX-Polypeptid überführbar ist, oder das als DNA-Sonde zur Hybridisierung für die Diagnose der Hämophilie B (Faktor IX-Mangel) brauchbar ist, wobei diese DNA im wesentlichen die folgende 129-Nuckleotid-Sequenz (gelesen in Reihen von 30 quer zur Seite)

(5') ATGTAACATG    TAACATTAAG    AATGGCAGAT
GCGAGCAGTT    TTGTAAAAAT    AGTGCTGATA
ACAAGGTGGT    TTGCTCCTGT    ACTGAGGGAT
ATCGACTTGC    AGAAAACCAG    AAGTCCTGTG
AACCAGCAG (3')

und/oder die dazu kompklementäre Sequenz umfaßt.

2. DNA-Molekül nach Anspruch 1, dadurch gekennzeichnet, daß seine Sequenz zu der der mRNA von humanem Faktor IX komplementär ist, wobei diese mRNA vom humanen Faktor IX-Gen transkribierbar ist.

3. Rekombinante DNA, dadurch gekennzeichnet, daß sie die DNA-Sequenz eines Klonierungsvektors und eine für den Vektor fremde DNA-Sequenz aufweist, wobei die fremde Sequenz eine solche nach Anspruch 1 oder 2 ist.

4. Wirtszelle, die mit rekombinanter DNA nach Anspruch 3 transformiert ist.

5. Wirtszelle nach Anspruch 4, in Form eines Bakteriums oder einer Hefe.

## Patentansprüche für den Vertragsstaat: AT

1. Rekombinantes DNA-Verfahren, wobei eine Fremd-DNA in einen Klonierungsvektor eingesetzt wird, dieser in einer Wirtszelle repliziert wird, die rekombinante DNA aus der Wirtszelle isoliert und gegebenenfalls die Fremd-DNA von der rekombinanten DNA, welche sie enthält, ausgeschnitten wird, dadurch gekennzeichnet, daß die Fremd-DNA den Vorläufer des humanen Faktor IX-Polypeptids kodiert, das in vivo durch Einwirkung eines Vitamin K-abhängigen Enzyms in das humane Faktor IX-Polypeptid überführbar ist, oder daß sie als DNA-Sonde zur Hybridisierung für die Diagnose der Hämophilie B (Faktor IX-Mangel) brauchbar ist, wobei diese DNA weiter dadurch gekennzeichnet ist, daß die Fremd-DNA im wesentlichen die folgende 129-Nukleotidsequenz (gelesen in Reihen von 30 quer zur Seite)

(5') ATGTAACATG    TAACATTAAG    AATGGCAGAT
GCGAGCAGTT    TTGTAAAAAT    AGTGCTGATA
ACAAGGTGGT    TTGCTCCTGT    ACTGAGGGAT
ATCGACTTGC    AGAAAACCAG    AAGTCCTGTG
AACCAGCAG (3')

und/oder die dazu komplementäre Sequenz enthält.

2. Verfahren nach Anspruch 1, dadurch gekennzeichnet, daß die Fremd-DNA komplementär zur mRNA des humanen Faktors IX ist, wobei diese mRNA vom Humanfaktor IX-Gen transkribierbar ist.

3. Verfahren nach einem der vorhergehenden Ansprüche, dadurch gekennzeichnet, daß die Wirtszelle eine Bakterium oder eine Hefe ist.

## Revendications pour les Etats contractants: BE CH DE FR IT LI LU NL SE

1. Molécule d'ADN (acide désoxyribonucléique) artificiel, produite par la technologique de l'ADN recombinant et codant en précurseur de polypeptide facteur IX humain qui est convertible dans l'être vivant ("in vivo") en le polypeptide facteur IX, par l'action d'une enzyme dont le fonctionnement dépend de la vitamine K, ou qui est utile à titre de sonde d'hybridisation pour le diagnostic de la maladie de Christmas, ledit ADN comprenant sensiblement la séquence des 129 nucléotides suivants (qu'on lit en rangées de 30 à travers la page):

(5') ATGTAACATG    TAACATTAAG    AATGGCAGAT
      GCGAGCAGTT    TTGTAAAAAT    AGTGCTGATA
      ACAAGGTGGT    TTGCTCCTGT    ACTGAGGGAT
      ATCGACTTGC    AGAAAACCAG    AAGTCCTGTG
      AACCAGCAG (3')

et/ou son complément.

2. Molécule de ADN selon la revendication 1, qui est complémentaire de l'ARN (acide ribonucléique) messager du facteur IX humain, ledit ARN$_m$ pouvant être transcrit à partir du génome du facteur IX humain.

3. ADN recombinant, qui comprend une séquence d'ADN comme véhicule de clonage et une séquence étrangère au véhicule de clonage, la séquence étrangère étant telle que revendiquée à la revendication 1 ou 2.

4. Cellule hôte, transformée à l'aide de l'ADN recombinant revendiqué à la revendication 3.

5. Cellule hôte selon la revendication 4, sous forme d'une bactérie ou d'une levure.

**Revendications pour l'Etat contractant: AT**

1. Procédé utilisant de l'ADN (acide désoxyribonucléique) recombinant, dans lequel de l'ADN étranger est inséré dans un véhicule de clonage, le véhicule de clonage est reproduit ("répliqué") dans une cellule hôte, l'ADN recombinant est récupéré de la cellule hôte et, éventuellement, l'ADN étranger est excisé de l'ADN recombinant le contenant, procédé caractérisé en ce que l'ADN étranger code un précurseur d'un polypeptide, facteur IX humain, qui est convertible chez l'être vivant (in vivo) en le polypeptide facteur IX humain, par l'action d'une enzyme dont le fonctionnement dépend de la vitamine K, ou qui est utile à titre de sonde d'hybridation pour le diagnostic de la maladie de Christmas, ledit ADN est en outre caractérisé en ce que l'ADN étranger comprend sensible la séquence des 129 nucléotides suivants (qu'on lit en rangées de 30 à travers la page)

(5') ATGTAACATG    TAACATTAAG    AATGGCAGAT
      GCGAGCAGTT    TTGTAAAAAT    AGTGCTGATA
      ACAAGGTGGT    TTGCTCCTGT    ACTGAGGGAT
      ATCGACTTGC    AGAAAACCAG    AAGTCCTGTG
      AACCAGCAG (3')

et/ou son complément.

2. Procédé selon la revendication 1, caractérisé en ce que l'ADN étranger est complémentaire de l'ARN messager du facteur IX humain, ledit ARN$_m$ pouvant être transcrit à partir du génome de facteur IX humain.

3. Procédé selon l'une quelconque des revendications précédentes, caractérisé en ce que la cellule hôte est une bactérie ou une levure.

```
1st amino acid              70                  75
      sequence :            Glu-Cys-Trp-Cys-Gln-Ala


    mRNA       :        5'  GA^A UG^U UGG UG^U CA^A GCN  3'
                            GA_G UG_C UGG UG_C CA_G GCN

Deoxyoligonucleotides  3'  CT^T AC^A ACC AC^A  GTT CG  (oligo N2A)
      synthesized  :        CT_C AC_G ACC AC_G

                       3'  CT^T AC^A ACC AC^A  GTC CG  (oligo N2B)
                           CT_C AC_G ACC AC_G


2nd amino acid              348                 352
      sequence :            His-Met-Phe-Cys-Ala


    mRNA       :        5'  CA^U AUG UU^U UG^U  GCN      3'
                            CA_C AUG UU_C UG_C GCN

Deoxyoligonucleotides
      synthesized :         GT^A TAC AA^A AC^A CG      (oligo N1)
                            GT_G TAC AA_G AC_G CG
```

## Fig. 1

1

EP 0 107 278 B1

Fig. 2

Fig. 3

Fig. 4

2

```
                              60
       E  S  N  P  C  L  N  G  G  M  C  K  D  D  I  N  S  Y
5'  TGAATCCAATCCATGTTTAAATGGCGGCATGTGCAAGGATGACATTAATTCCTAT
         10        20        30        40        50


     70                             80                          90
      E  C  W  C  Q  A  G  F  E  G  T  N  C  E  L  D  A  T  C  S  I  K
    GAATGTTGGTGTCAAGCTGGATTTGAAGGAACGAACTGTGAATTAGATGCAACATGCAGCATTAA
         60        70        80        90       100       110       120


                100
     N  G  R  C  K  Q  F  C  K  R  D  T  D  N  K  V  V  C
    GAATGGCAGATGCAAGCAGTTTTGTAAAAGGGACACAGATAACAAGGTGGTTTGT
        130       140       150       160       170


   110                             120                         130
    S  C  T  D  G  Y  R  L  A  E  D  Q  K  S  C  E  P  A  V  P  F  P
   TCCTGTACTGACGGATACCGACTTGCAGAAGACCAAAAGTCCTGCGAACCAGCAGTGCCATTTCC
       180       190       200       210       220       230       240


                               140                          150
    C  G  R  V  S  V  S  H [V  R  P  R  F  H  G  L  C  S  C  *  E ]
   CTGTGGACGAGTCTCTGTCTCACAT GTGAGGCCCCGCTTTCACGGTCTGTGTTCGTGCTGAGAA  3'
       250       260      [ 270       280       290       300    ]
```

Fig. 5

3

Fig. 6

EP 0 107 278 B1

```
GAATTCCTTGTGCCATTATTTTATTTCTGGAATCTTCAGCCTTTTAGCTGAGGGCAAAAGATTGCTGATTAGGAAGCAATATTTCCCACCTCCTGCGCAAAACAAGCCAAAGATCAACAG

CAGCAGCAACATACTGAGCCCTAAAG-GGTGACAAATTTGGAGAATGATACAGAGGTCTGGTTACTTCTTAGCCAATGACACAGAATCACAATTGAGAAAACACAGAGTTTATTCATTCC

CATTGTGCATGCCCTGACAAACCAAGCTGCACCTTTCGTAACTTATCACAATCTCATATTGACGGAACACTTTCTACAGGTAATGTTTAGTTTGGCTGAACACTTTAGCAATTGCTTCTG

TAGCAACAAAATGATAGCTAGTAACAGAAAAGTTCAGGAATATTACCACTGTTAGTGAGGAGAAAGGCCTTTTAATTAATTAATTAATTAATTAATAGAACCAAGTACCATCTTTTTG-A

TCATGCCCTTAGTGAATTATTGGTAGCAAAGGTTAAAGCTCAAGCTGGTTCCTTTGTCCCCTG-CAACAGTTGATTT-CCTCCCTTTATCTCCTGAAGTACCGTAAG-ACTAAGAGCCAA

TTATTACATTTG-TCATGTCAGCTATATGTAAAATAGAGTTTAAAAGTTTAGATTCATCACTCAAAAATTCATATTCTCCAAAACCATACAGTCACTCTGTTAGCCTGTGTTCCCCCAGA

AAAAAAGTCACAAGCTTATTTATTAACATGTGCAATCCCA-GGGGCAAGAGAAAGGAACTGAAGAGTGAGGCAGAAAGGAAAAGAAAG-CAATAAGAGGATGAGTTATCAAACTACTCGT

TTCTAACAGCAACTGATTGCTTAACTTCCTAGGACTGTCTCCAATAAGTCAAATTG-CCTCAGGTTAGCCACCTGAGTGGAAAGAAGCGGTGAAAGAATTTGTCTGTCAGTATCTGTCTC

TCATTGGTTAGAAGTTCGACTTATGGGGAATTAACTC-CTCACATTTCCTAGTTGGATATGCTTGGGTACAGAGGGT-ATAGTAGCATTACTGCCTCA-GCATGAACAGGGAAGCTTTCA
```

FIG. 7a

EP 0 107 278 B1

```
AGGCAAAAGACACATAGTGCAGCTATGAGCCAAGGCAATTCAAGGATACACCCATAGGAGGCTGGTTGACATCCACCCAGAGCTAATCACCACCATGCTGGAAAAAGACACAGGTGAAGC
  1090      1100      1110      1120      1130      1140      1150      1160      1170      1180      1190      1200

TGAGAAGAATGAAGGTGGTGCATAGGAGGTATCTAATACAGTCACTCATTTTCAAACTTTCCATGTTATGATTGCACTGACCACTGAGGATTTCTATTGAAAGTTTTACTGTTGTCAAAC
  1210      1220      1230      1240      1250      1260      1270      1280      1290      1300      1310      1320

ACGTACACAAGGGGAAAGGTGTCTTACATTGTTTATGTTCCTGTGCTGCTCTAGAAACAGAAATAGGCTCAAGGCAGAGCCTGTTTTTCTTAATTCAGCAGGTCTAAGCTAACAAGTCCT
  1330      1340      1350      1360      1370      1380      1390      1400      1410      1420      1430      1440

GAAACATGGTACTTCCTGTTATTGGTATTGCATAGGAGAAACAAAGGGAAAGCACAGTAATTAGAAAATACAAAACAAGATGGCAGGAATAAGCCAAAAATATCAGGAAACACAATTATT
  1450      1460      1470      1480      1490      1500      1510      1520      1530      1540      1550      1560

GTGAATTGGGATTAAACTAATCTATTAATAATGACAACTTTCAGCTTGGAGTTAAAAATTTAATTGTATACTGTTAACGAAAGTGATACCTAAAATAAAATTACACTGGGAGGCCAAAAT
  1570      1580      1590      1600      1610      1620      1630      1640      1650      1660      1670      1680

GAAGGGATGTGAAAAGAACTATCAGGTAAAAACTAACAAAAAGAAACTAGCAAAGCAATCTTAATATCAGACAAAATAGATTCAAGAGGAAAATCATTTCAAAAGACAAGAGATTTTTTT
  1690      1700      1710      1720      1730      1740      1750      1760      1770      1780      1790      1800

TATTAATAAGGGGAATTGCATAGGAGAGTAAAGAAAATGTGGGCCACTGGAATGCTTAGCACTAATGACATATTGGTCTTTGGTCTTCAGTTACCTTACAGGACCCTATTTCATTCTCTT
  1810      1820      1830      1840      1850      1860      1870      1880      1890      1900      1910      1920

ATGTTTGATATGTAACCACCTCAGCCAGCTTCAAGTTGCTTTTTGGCCCTAATGGACTTCCTAGCACTATAATTTCTTTTTTTTTAAATGTTTTATTTTAGGTTTAGGGGTACATGTGAA
  1930      1940      1950      1960      1970      1980      1990      2000      2010      2020      2030      2040

GGTTTGTTACATAGATAAACATGTGTCACAGGGGTTTGTTGTACATATTATTACATGACGCAGATATTCAGCTCAGTACCAAATAGTGATCTTTTCTGCTCCTCTGCCTCATCCCACCCT
  2050      2060      2070      2080      2090      2100      2110      2120      2130      2140      2150      2160
```

FIG. 7b

EP 0 107 278 B1

CCTCCCTCAAGTAGACTCCAGTATCTGTTGTTTCCTTCTTTGTGTTTATAAGTTCTTAACACTTAGCTCCCGCTTACAAGTGAGAACCTGCAGTATTTGATTTTTGTTCCTACGCTAGTT

TCCTAAGGATGATAGCCTCCAGCTCCATTCATATTCCCACAAAAGACATAATCTCCTTCTTTTCTATGGCTGCATAATATTCCATGGTATATATGAACCACATTTTCTTTATCCAGTCTG

TCATTGATGGGCATTTAGGTTGATTCCATGTCTGCTATTCTAACACTGTAATTTCTAAAGACTTCCAGATTCTACTTTTATAGGTAACCTGTTAAACAGTCTAGCTCTGGAAGCCAAGCA

ATTTCTAGAATAACTAAGCAATAGAAATTACACTTCAATGCAGAAAGGCAGTATCTACATGAGATTATGAAATTGCGGTTGCTTTTTGTGTTCACTGAAAAAAATAAGTAAAACTGTAAC

TTTCAGAAAAAATGATTGTACATATAGAAAACCCAAAGCATCTAAACAATTAAAATAAATAAGTATAGAAAGATTACTGGATACAGAGTCAACATACAAATATCAATTGTATGTCTATAT

ACCAGCAACGATTCAAAAATGATTTTTATAATAGCATTAAAAATTAGACGCTTAGTAATAAATGTGAGAAAGATGTGCAAGAACTCTACATAAAAAATTATGAGACGTTATTGAGAAAAA

TTAAGGAAAACCTAAATAAATGAATAGGCAATGTTTATCAATTAAAGGATACAATATAGTAAATATATCAAATGTTTACTAATGGATTCAATGCAATACCAAAGGTCCCAGCAGGCTTTT

TTTGGTGGTGGGAGGTCGGGCAGGATTCATAAGCTAATTATAAAATGCATATGGAAATGCAAAGAGCCAAGGATAGCCAAGACAGTTTTGAGGAAGAATAAACTTGTACTACTTACACTA

CCAGATGTCAAGACTTATTATCGAGTTACATTTATTAAGACAGTGTGGTACTGACACAAGGATAGACAAATAGATCAGTGAAACACACTAGAGTGCTCAGAAGAAGCACACCTGTACATA

TATAAAGGCTTGATTTATGATAGAGGTGCCAGTGCAGTAGAGAAGGAAATTATTGGTGTTTTCAATAAAAAGTGATAGGTCAATTAGATATTCATATGGCATGAAGTATGAAACAATAAC

FIG. 7c

EP 0 107 278 B1

AATTTATATTCATAACTTGCAGAAAGCAAAAATTTCTTAAAATACAAAAAGTGATCACCATAAAGGAAAAGATTGATAAACTGGACTATATTAAAACTAAGGACTCCTGTTCAGCAAAAG

ACACTACTTCGACTGAAAAGACAAGTCACAGAGTGAGACAAGATATCTGCAATACAGATACCTAATAACTGAACCCCATACAGTGATGGTGGGAATTTAAGTTCGTACAATCATTTTAGA

AAATTGCTTGGCAGTAATCTACTAGATCTGAACATGTGATCCAGTAATTACACTCATAATTATAAGCCAGTAAAAAGGCATGTTTATGTCACCAAAAGATATATACAAGAATGTTCATTA

CACTATTATACATAAGAGCCAAAAACTGGAAACAAACCAAATATCCATTAACAGTAGAATGAATAAATAAAAGCTGTAATAGTAATACAGTGGAATACTACACAGCAATGTAAATGAACT

ACTGCTGTACAAAACAACATGGTTTAATCTCACAGACAAAATGTTAAATGAAAGACACAGACGAGTACATATTGCGAACTTCTGTTTATAATTCAAGAACTGGCAAGAACTGTTTACTGT

GTTAGAAGTCCAGGTAATGGTAACCTATAAAAAGGAAAAAGGGTGGAATGATTGGGAGGGGGCATCTTCTGGGGTATTGATAATGTGCTATGTATTGGTCAGTTTAGTGTTTAAACAGGC

TCATTTACTTTGTGAAAACTTACACTAAAATTGTGTGTATTTTTTGAATATATGTTATACATTAATAAATAGGGTTTTTAAACCTGTAGTTCATAATTTAGTGAAAGTAGAATATCCAAA

CATTTAGTTTTAAAACCAATCAATTATAGTGCTACCATCATTTTTATGCATTATTGAGAAGTTTATTTTACCTTTCTTTCCACTCTTATTTCAAGGCTCCAAAATTTCTCTCCCCAACGTA

TATTGGGGGCAACATGAATGCCCCCAATGTATATTTGACCCATACATGAGT^OCAGTAGTTCCATGTACTTTTTAGAAAATGCATGTTAAATGATGCTGTTACTGTCTATTTTGCTTCTTTTA

D   V   T   C   N   I   K   N   G   R   C   E   Q   F   C   K   N   S   A   D   N   K   V   V   C   S   C   T   E   G   Y   R   L   A   E   N   Q   K   S   C
GATGTAACATGTAACATTAAGAATGGCAGATGCGAGCAGTTTTGTAAAAATAGTGCTGATAACAAGGTGGTTTGCTCCTGTACTGAGGGATATCGACTTGCAGAAAACCAGAAGTCCTGT

FIG. 7d

E  P  A

GAACCAGCAGGTCATAATCTGAATAAGATTTTTTAAAGAAAATCTGTATCTGAAACTTCAGCATTTTAACAAACCTACATAATTTTAATTCCTACTTGAATCTGCTTCCTTTTGAAATCA

TAGAAAATATCAGTAGCTTGAATTAGACCAATTAATTTTCTAGATTGCATCATATTTTAAATATAAACTATGTAATCATCTACAACCTGAATTCTTTCTGAGTCCAATTTGTCCAATTTT

TTTCTCTAACATTTATATCACAAAGCAATTAATTTGTGTGATTTCTGCATATGTATTTGTAATTCATCAAGTCAAATCAATGTAGTAATACTATATCATAAAATATACACAAATAATTGA

GTGATAGGCTTCTAGTATAAGGACGGTAAGTTTGAAGCATGATTCTATCTGGGCTGGCTAGTTTACTCTGAGAAAGTTATTTTTTATTGTTGGGTCTTAAGCTGAGTTTACACACTTGGT

GTCAGAATGATTCCGGCAATGAACTGTTTTATGTTCTGCTAGGCTGATCAGCACAATCTATATGGCTGTGAACAAAACAATGTTTCCCAGTCATACCAACCATGCCACCATTTTAACAGC

TGATTAGTGTATTCAGAACATCTCCACTCCATGTTCGTATGGCTGTTATCTAAAGATGAAAGCAGTAGACACTTTTATTTTTTGAAAAATTTAGGCTCTGCAGGGTCAATTATATTTGAT

AAATGAGGGGCTTTTTTTGAAGCAAACTAGATATAATTTCTTTTGCATTTCTAAAGCCTGATATCTTATTAATTGGTACATTAAATTGTGCACCATTTCTCTGTAACTGTTTCAGTACCTG

TCTCAGCACTATACCAGGCAGAAGAAATTAAAGAAAAGAACCAGTGCCGAGATCAGCTTGGTCAGGGAGACCCTAATCCTGCGGCACTAGAGGAATTAAAGACACACACACAGAAATATA

GAGTATGAAGTGGGAAAATCAGGGGTCTCACAGCCTTCAGAGCTGAGAGCCCCGAACAGAGATTTACCCACATATTTATTGACAGCAAGCCAGTCATAAGATTTACTGAAAGTATTCCTTA

TGGGAAAATAAAGGGATGAGTCGGCTAGTTATCTGCAGCAGGAACATGTCCTTAAGGCACAAATCACTTATGCAATTGTCTGTGGTTTAAGAACACCTTTAAGCAGTTTTCCGCCCTGGGT

FIG. 7e

10

GGGCCAGGTGTTCCTTGCCCTCATTCTGGTAAACCCACAACCTTCCAGTGTGGATATCAAGGCCATCACGAGCATATCACAGTGCTGCAGAGATTTTGTTTATGGCCAGTTTTGGGGCCA

GTTTATGGCCAGATTTGGAGGCCTGTTCCCAACAAACCAGAAGCTAGGAATATATATCCTGCAAATAAAATGAAGAATCTCTAAGGCTTCGGGCCTGCCCACTTGTTCTTCTGCCTGGTT

CTTCACATACACTGTCTCAAAGCTAGTCTACCTTGAGAGGAGCATGAATATGTGTGTGGGTGTGTGTCTGTGTATTTTAACCTTAAAAAACCTAACTTCCAGTATAGACAGATGGCATACT

AGCTAAACCCTTACAAGTTCTTCTATGCTATAAAAGAGAAACAGAATTGAGAACCACCTCCAACTATTAAGTGTTATATTTGAATATAGCCTTAGCTTTAGCAGAATAAGTAGGCCAAAC

TTAAAATAAGCTTTTCTGCCTTTTCAATGATAAAGGTCCCTTTTCTGTAGCCATTGTTGATTGTGTACACTTATACATAAGTATTTTGAACTAATTTCCTGTTTTCTCAACCACTTGCTG

TCTTCATGATACTTTGTCGCAGCTGGTTGCTATAGAAATGTCTGTTACAAGGAATGTGGCTTGAAGGAAAGTGATAAATGAAAATGAAATGTGAAGTGACTTTGTTTGACTACAAATTCC

CATTCTGGTAGTCCCCAGTGTATCAATACATTATTTTTCTTTAGAAAATAAACCAACCCAAGGAAAAATGGTGGGCAGGTCCTGGTGAATATGGCTGTGATAATTATATTAGCAATCTCT

TTGGCTAATATTTGAAGCCCAAATAATTGAATCACAATGATCTCTCCCCAGAAAATATATAAAATGCACCTTGGAATCTAGAAGGCCTTTTAGTCTGCAAAAGAAACCTTCTTAATCATA

AGCAGCAGAAGTCCCATTTACCAAATTGGAAAGTTAAAGTTACAAAGCATCAATCATCAGACTTCCATTCAGGGATGGCAATTGGGAGTAAGACTTTTTAGTAAAGAAACTAAACACAAA

FIG. 7f

EP 0 107 278 B1

```
GTCATTAGACTCTGTAAAAGTCTTACCAAATTTGATTCTGGAACACCTATTCTATTTCCGTAAAGATGATGAATTCCGGAGCCAAATGTTCTTTTCATGAAGGATTTGAAAACTGTCCAT
    6850      6860      6870      6880      6890      6900      6910      6920      6930      6940      6950      6960


GAAAATAACGCAATCAACCTTTTAGCTTGAGACTCTATTCACTGATTAGATTTTTTTAAATACTGATGGGCCTGCTTCTCAGAAGTGACAAGGATGGGCCTCAATCTCAATTTTTGTAAT
    6970      6980      6990      7000      7010      7020      7030      7040      7050      7060      7070      7080

                                                         J'
                                                      Iv  P   F   P   C   G   R   V   S   V   S   Q   T   S   K   L   T   R   A   E   A
ACATGTTCCATTTGCCAATGAGAAATATCAGGTTACTAATTTTTCTTCTATTTTTCTAdTGCCATTTCCATGTGGAAGAGTTTCTGTTTCACAAACTTCTAAGCTCACCCGTGCTGAGGC
    7090      7100      7110      7120      7130      7140      7150      7160      7170      7180      7190      7200


    V   F   P   D   V   D   Y   V   N   S   T   E   A   E   T   I   L   D   N   I   T   Q   S   T   Q   S   F   N   D   F   T   R   V   V   G   G   E   D   A   K
TGTTTTTCCTGATGTGGACTATGTAAATTCTACTGAAGCTGAAACCATTTTGGATAACATCACTCAAAGCACCCAATCATTTAATGACTTCACTCGGGTTGTTGGTGGAGAAGATGCCAA
    7210      7220      7230      7240      7250      7260      7270      7280      7290      7300      7310      7320


    P   G   Q   F   P   N   Q
                          J"
ACCAGGTCAATTCCCTTGGCAGGTACTTTATACTGATGGTGTGTCAAAACTGGAGCTCAGCTGGCAAGACACAGGCCAGGTGGGAGACTGAGGCTATTTTACTAGACAGACCTATTGGGA
    7330      7340      7350      7360      7370      7380      7390      7400      7410      7420      7430      7440


TGTGAGAAGTATTTAGGCAAGTTTCAGCACTAACCAATGTGAGAAGGCCTCCAGAGATGAGCAGTTGGTGAAAGAGAGGCTCAAAACCAGCTACCATACAGGTCAAGAAGAATTTGGCAT
    7450      7460      7470      7480      7490      7500      7510      7520      7530      7540      7550      7560


TAAGGAAACAGCATAGCAGGATTCCAGACAGGCA---GGTCAACAACATGAAGGTCTGGAAGAAAGGTCGCAGGTACTCAGGTTCAGGGCACTACTTCAGCTTCAGCCCTTGCAAAAACT
    7570      7580      7590      7600      7610      7620      7630      7640      7650      7660      7670      7680


GGTGAGAGTTGGAAAGTCTTTAGGCTAAGAAAAATTGGATTATTTAAAAGGGGGTAAAGAAAGGGACTCAAGGAGGAAGGATTAAGGCAAGAACTAGGTT-CAAGAACAGGGCATGAGAG
    7690      7700      7710      7720      7730      7740      7750      7760      7770      7780      7790      7800


AGAGTCTTGATCTACCACTATAGTTCTCGT-------------------------------------------------------------------------GGAACTGAACGGAGATTACT
    7810      7820      7830      7840      7850      7860      7870      7880      7890      7900      7910      7920


TAACCGA-ATTTGA-AAC-CCTGG-CAACACG-CGAACCCCACCTCTAATTAAAAAAAATACAAAATTAGCTAGGTGTGATGACTCCCACCTGTGCTCCCAGCTATTCAGGAGGCTGAGG
    7930      7940      7950      7960      7970      7980      7990      8000      8010      8020      8030      8040
```

FIG. 7g

EP 0 107 278 B1

```
TGGGAGAATCACCTGAGCCTGGAAAGTCGAGGCTGCAGTGAATTGTGATCACACCACTGCACTTCAGCCTGAGTGACAGAGTAAGACCCTATCTCAAAAAACAGAAAAAGAAAAACACTG
     8050      8060      8070      8080      8090      8100      8110      8120      8130      8140      8150      8160


GCCCAAAGGAAATGAACTTGTTACAGAAGCCGGGGTTCAAAACACCAAATAATGCACTTGTACCTAGTCCTTCCCGGGTGCTCTGCAGACATTTCTCCAAGCGTAGTCTGCAAACAACCT
     8170      8180      8190      9200      8210      9220      8230      8240      8250      8260      8270      8280

ACATATGTAGAATTACCTATGCACATTTTTCATTTAACAACCAAG-GCTACATTTGTAGCAAAATCTGGGTTGTAACTTAGCCTACAGCTGAAGCCTAAGAGATTCCGTCTGTGAGAAGA
     8290      8300      8310      9320      8330      8340      8350      8360      8370      8380      8390      9400

AATAACCCACCTCTTTGGCCCCCCTCCCCAGGCAGGAAGCCAGGATGGTCCTTATATAAAGTTGTGCTGT-CAATAGGTAACCACTAGCCACATATG--TTTAAATTTAAATTAACTACA
     8410      8420      8430      8440      8450      8460      8470      8480      8490      8500      8510      8520

ATTAAGAGAAATTAAAAATTCAATTC-TCAATTGCACCTGCCAAATTTTAAGCACATAACAACCACATGTGG-TAGTAACTACTGTATTGGAGAGTGCAAGCGGAGATAGAACACTCTAT
     8530      8540      8550      8560      8570      8580      8590      8600      8610      8620      8630      8640

TACTGCAGAAATTTCTATTGGATAGCACTTATAATAGTTTAGTGTAACTTAAAACT-CCTAGTTGCCACAAGTCATGATTTAGTAGTAATTTCATGGA------------------------
     8650      8660      8670      8680      8690      8700      8710      8720      8730      8740      8750      8760

---------------------------------------------------------------------------------------------------------------------
     8770      8780      8790      8800      8810      8820      8830      8840      8850      8860      8870      8880

---------------------------------------------------------------------------------------------------------------------
     8890      8900      8910      8920      8930      8940      8950      8960      8970      8980      8990      9000

---------------------------------------------------------------------------------------------------------------------
     9010      9020      9030      9040      9050      9060      9070      9080      9090      9100      9110      9120

--------------------------------------------------------------------------AAAAGACAATATTTGCTG-ACCGATCTTATAACTCATAAATG
     9130      9140      9150      9160      9170      9180      9190      9200      9210      9220      9230      9240
```

FIG. 7h

```
G-ACACTGTATGTTCCTTTTTACCTCCTCTGTTCTACTTAATTGCACCCTATGAGGACTGCTTCCCTTACCTACCATAAACCCTTCCTTCACTCATCCATATCTTTACTCTTCTTCACA
   9250      9260      9270      9280      9290      9300      9310      9320      9330      9340      9350      9360

ACTCTGTAATATTGACCTTCTTTA-GAACCTTTCCTGGAACAATCCCTCTTAAGTGCAAGCACTGTTATTATGCCTTCAATGTATTTAATATCCATGTATCTATTCTCTCTAATTTTGTC
   9370      9380      9390      9400      9410      9420      9430      9440      9450      9460      9470      9480

ATTTTGTGTTCTCATGTATTTTCATTCATTATGTGTCCAACTTCCATGGATAACATGGTTACAACAAAAGATCCTACTTTATGACAATTATCTTCCTTGGGTTTGTGGGACATAGAAACAG
   9490      9500      9510      9520      9530      9540      9550      9560      9570      9580      9590      9600

TGCTCAGAGTAGGGGATCCAAGAACCCAGGAGAATATATTAGCTAAGAAGATAAACTTCCGTTTTAAAAAGTCCAAGATTCAGGAGATCAAAACCATCCTGGCTAACATAGTGAAACCCCG
   9610      9620      9630      9640      9650      9660      9670      9680      9690      9700      9710      9720

TCTCTTCCAAAAATACAAAAAATTAGCCCCGGCGTGGTGGCAGGCGCCTATAGTCCCAGCTACACGGGAGGCTGAGGCAGGAGAATGGCGTGAACCGGGGAGGCGGAGGCTGGCAGTGAGCC
   9730      9740      9750      9760      9770      9780      9790      9800      9810      9820      9830      9840

GAGATCCCGCCACTGCACTCCAGCCTGGGCGACAGAGCGAGACTCC---AAAAAAAAAAAAAAAAAAAAAAAAGTGT-TGT-TTGTGAGTTT--
   9850      9860      9870      9880      9890      9900      9910      9920      9930      9940      9950      9960

-------------------------------------------------------------------------------------------------
   9970      9980      9990     10000     10010     10020     10030     10040     10050     10060     10070     10080

-------------------------------------------------------------------------------------------------
  10090     10100     10110     10120     10130     10140     10150     10160     10170     10180     10190     10200

---CCCTATTCAACCACATGAGATTACTGATGTGACAGATTCAAGCACTTTATCTTTCCAAAGGCAAGAAGCTGAGCTACTTTCCAGAATAGTTGTGAAAGACCCTGTCAT
  10210     10220     10230     10240     10250     10260     10270     10280     10290     10300     10310     10320
```

FIG. 7i

13

14

ACTTCTGCATTGTTTUCTCCACACCACCTCCATCCAGTTCCTTATGAATGGTTACTGGTTTTCAAAAATATGAGATAAATTGAGTGTATAAAAGTCATTTTTAGACAAAATGAAACAGGA
10330      10340      10350      10360      10370      10380      10390      10400      10410      10420      10430      10440

AATGAAAGAAACCAGAATCTCTCCTCATTTGTGGATGGGCCAGCTCCACCATGTCATGGTTAATCTGCAGGGAGGAAATACTAGATTTGATTGCAGATCAGACTGCAGCAAACCTGCTGT
10450      10460      10470      10480      10490      10500      10510      10520      10530      10540      10550      10560

GACTAAGGCATCAAGAGAAAGCAAGCAACAGCTGGGGCTTCAGTGGTGAAAACATTATATATCTAGCTTTGAATATGAAATACTGTTTAGCAGTGTCACCTAGAAAAGAGTGTTTCAAAA
10570      10580      10590      10600      10610      10620      10630      10640      10650      10660      10670      10680

TGCTGATGCAACCTTTCTCTTCAGAGTTGTTTCTTTTATCTTTCAAATTTAGCCAGGGTGGGAAATAAAGTGATCACTTGGTGAAGAAATCTCACAAAGAAGAACATAGAGAGTTCACTT
10690      10700      10710      10720      10730      10740      10750      10760      10770      10780      10790      10800

TCATCTGGAGTAATGAACAGATTGAACAAACTAGAAATGGTTAGTCTGTTAAAGAAAAGGTGTAGGTGAGCTGTTTGCAAGAGCCACAAGGGAAAGGGGAAGACAACTTCTTTGTGGACT
10810      10820      10830      10840      10850      10860      10870      10880      10890      10900      10910      10920

TAAGGGTGAAAGTTGCAAGCAGGCAAGACGATTCTGACCTCCATTAAGAAAGCCCTTTCCAACCAACAACCACTGGGTTGGTTACACAGGTTGGGCAGCATTGGGAGCAAATGTTGATTG
10930      10940      10950      10960      10970      10980      10990      11000      11010      11020      11030      11040

AACAAATGTTTGTCGGAATTGTTGACTTAAAGAGCTGTTCTGTCACTGGGGACAGCGGCTAGATAGCCCCATTCAGGGAG-GGGCATTTGTTCACCTGGCCAGAGATCAGAGCAGGCTAA
11050      11060      11070      11080      11090      11100      11110      11120      11130      11140      11150      11160

GG-ACT-CTGGATCCTGTCCAGCTTTGAGACCCTACAGAGAGCCATGTTCTCCTAGCACGTATCCCGTCTGCGGTCACGGTCATTTCTTACCTTATTCCAGGGCTTTCACCTCAGCTTGCCA
11170      11180      11190      11200      11210      11220      11230      11240      11250      11260      11270      11280

GGCTGGAGCCAAGGGCAACGCAGCCGC-CTTGTTCGCGATGGTAGCTTCCCAGGAGCCCCCTATGGTTCCGGAACGGCGCTG--CCCATCCTGTTTGCTACCTCCTAAAGCCAAAGG--C
11290      11300      11310      11320      11330      11340      11350      11360      11370      11380      11390      11400

TGGCGGG-C-GG-C---CTTCTAAAGTCGCGCAAGGTTAGAAGGTTCCGGACAGGAACGGCGTGAGGCCAATGGAAGGAGGTACTTCAGTTTCCCTCCAGGCCCGCGCGATGGGCTCAGA
11410      11420      11430      11440      11450      11460      11470      11480      11490      11500      11510      11520

FIG. 7j

GCTCCTTGAGAACTCGGGAAAGGAAGCAGGGTCTCTGAAGAAATACTTCAGGAGTAGAAAGAGGAAGCTAGAGGGTTAAATGCACTACACAGGAACAGAAATGAGTTTTTCTTAGAGTTA
11530      11540      11550      11560      11570      11580      11590      11600      11610      11620      11630      11640

GTATATGTCTAGAGGTGTAGTAAACTAAAACAAGTCTTGAATTGCATACCGCCACGTAGGGAAGAAATGAAAACCTTTGAATATTAGTGAAAAAAGGGAAACTGCAACGCCTGTATTACT
11650      11660      11670      11680      11690      11700      11710      11720      11730      11740      11750      . 11760

AGATAGCTTTCATCAACAGCTCAAAACCGACAGATTTAAAGAAGCAACACCGCATTTTGGCTTTCTAAAGCTTTAATTTGGTTTGGATCCCATGCCCATGACCCTGCCAGCTG

11770      11780      11790      11800      11810      11820      11830      11840      11850      11860      11870

FIG. 7k

FIG. 8(a)

| | | | |
|---|---|---|---|
| → 1 | 0.000 | ECOR1 | GAATTC |
| 30 | 0.002 | HINF1 | GAATC |
| 33 | 0.003 | MBO11 | TCTTC |
| 46 | 0.004 | ALU1 | AGCT |
| 48 | 0.004 | DDE1 | CTGAG |
| 50 | 0.004 | MNL1 | GAGG |
| 89 | 0.007 | MNL1 | CCTC |
| 94 | 0.008 | MST1 | TGCGCA |
| 95 | 0.008 | HHA1 | GCGC |
| 112 | 0.009 | MBO1 | GATC |
| 120 | 0.010 | BBV1 | GCAGC |
| 120 | 0.010 | FNU4H1 | GCAGC |
| 123 | 0.010 | BBV1 | GCAGC |
| 123 | 0.010 | FNU4H1 | GCAGC |
| 134 | 0.011 | DDE1 | CTGAG |
| 148 | 0.012 | HPH1 | GGTGA |
| 173 | 0.014 | MNL1 | GAGG |
| 188 | 0.016 | DDE1 | CTTAG |
| 204 | 0.017 | HINF1 | GAATC |
| 247 | 0.021 | SPH1 | GCATGC |
| 265 | 0.022 | ALU1 | AGCT |
| 266 | 0.022 | BBV1 | GCTGC |
| 266 | 0.022 | FNU4H1 | GCTGC |
| 305 | 0.026 | XMN1 | GAACACTTTC |
| 376 | 0.032 | ALU1 | AGCT |
| 417 | 0.035 | MNL1 | GAGG |
| 425 | 0.036 | STU1 | AGGCCT |
| 426 | 0.036 | HAE111 | GGCC |
| 465 | 0.039 | RSA1 | GTAC |
| 488 | 0.041 | DDE1 | CTTAG |
| 517 | 0.043 | ALU1 | AGCT |
| 523 | 0.044 | ALU1 | AGCT |
| | | | |
| 559 | 0.047 | MNL1 | CCTC |
| 578 | 0.049 | RSA1 | GTAC |
| 590 | 0.050 | DDE1 | CTAAG |
| 621 | 0.052 | ALU1 | AGCT |
| 652 | 0.055 | HINF1 | GATTC |
| → 732 | 0.062 | HIND111 | AAGCTT |
| 733 | 0.062 | ALU1 | AGCT |
| 781 | 0.066 | MBO11 | GAAGA |
| 788 | 0.066 | MNL1 | GAGG |
| 816 | 0.069 | MNL1 | GAGG |

FIG. 8(b)

| | | | |
|---|---|---|---|
| 818 | 0.069 | FOK1 | GGATG |
| 898 | 0.076 | MNL1 | CCTC |
| 898 | 0.076 | MST11 | CCTCAGG |
| 899 | 0.076 | DDE1 | CTCAG |
| 913 | 0.077 | DDE1 | CTGAG |
| 929 | 0.078 | HPH1 | GGTGA |
| 976 | 0.082 | TAQ1 | TCGA |
| 1027 | 0.086 | RSA1 | GTAC |
| 1032 | 0.087 | MNL1 | GAGG |
| 1054 | 0.089 | MNL1 | CCTC |
| 1072 | 0.090 | HIND111 | AAGCTT |
| 1073 | 0.090 | ALU1 | AGCT |
| 1099 | 0.092 | BBV1 | GCAGC |
| 1099 | 0.092 | FNU4H1 | GCAGC |
| 1101 | 0.093 | ALU1 | AGCT |
| 1138 | 0.096 | MNL1 | GAGG |
| 1145 | 0.096 | HINC11 | GTTGAC |
| 1150 | 0.097 | FOK1 | CATCC |
| 1161 | 0.098 | ALU1 | AGCT |
| 1167 | 0.098 | HPH1 | TCACC |
| 1193 | 0.100 | HPH1 | GGTGA |
| 1198 | 0.101 | ALU1 | AGCT |
| 1200 | 0.101 | DDE1 | CTGAG |
| 1204 | 0.101 | MBO11 | GAAGA |
| 1226 | 0.103 | MNL1 | GAGG |
| 1284 | 0.108 | DDE1 | CTGAG |
| 1286 | 0.108 | MNL1 | GAGG |
| 1323 | 0.111 | RSA1 | GTAC |
| 1365 | 0.115 | BBV1 | GCTGC |
| 1365 | 0.115 | FNU4H1 | GCTGC |
| 1370 | 0.115 | XBA1 | TCTAGA |
| 1424 | 0.120 | DDE1 | CTAAG |
| 1427 | 0.120 | ALU1 | AGCT |
| 1449 | 0.122 | RSA1 | GTAC |
| 1603 | 0.135 | ALU1 | AGCT |
| 1626 | 0.137 | ACC1 | GTATAC |
| 1633 | 0.137 | HINC11 | GTTAAC |
| 1633 | 0.137 | HPA1 | GTTAAC |
| 1670 | 0.141 | MNL1 | GAGG |
| 1672 | 0.141 | HAE111 | GGCC |
| 1685 | 0.142 | FOK1 | GGATG |
| 1759 | 0.148 | HINF1 | GATTC |
| 1766 | 0.149 | MNL1 | GAGG |
| 1841 | 0.155 | SAU961 | GGGCC |
| 1842 | 0.155 | HAE111 | GGCC |

FIG. 8(c)

| | | | |
|---|---|---|---|
| 1855 | 0.156 | DDE1 | CTTAG |
| 1884 | 0.159 | MBO11 | TCTTC |
| 1901 | 0.160 | AVA11 | GGACC |
| 1901 | 0.160 | SAU961 | GGACC |
| 1939 | 0.163 | MNL1 | CCTC |
| 1940 | 0.163 | DDE1 | CTCAG |

.

| | | | |
|---|---|---|---|
| 1947 | 0.164 | ALU1 | AGCT |
| 1965 | 0.165 | HAE111 | GGCC |
| 1965 | 0.165 | SAU961 | GGCCC |
| 2030 | 0.171 | RSA1 | GTAC |
| 2081 | 0.175 | RSA1 | GTAC |
| 2097 | 0.177 | HGA1 | GACGC |
| 2110 | 0.178 | ALU1 | AGCT |
| 2112 | 0.178 | DDE1 | CTCAG |
| 2116 | 0.178 | RSA1 | GTAC |
| 2128 | 0.179 | MBO1 | GATC |
| 2141 | 0.180 | MNL1 | CCTC |
| 2147 | 0.181 | MNL1 | CCTC |
| 2150 | 0.181 | FOK1 | CATCC |
| 2158 | 0.182 | MNL1 | CCTC |
| 2161 | 0.182 | MNL1 | CCTC |
| 2165 | 0.182 | MNL1 | CCTC |
| 2171 | 0.183 | ACC1 | GTAGAC |
| 2174 | 0.183 | HINF1 | GACTC |
| 2222 | 0.187 | DDE1 | CTTAG |
| 2225 | 0.187 | ALU1 | AGCT |
| 2248 | 0.189 | PST1 | CTGCAG |
| 2282 | 0.192 | MST11 | CCTAAGG |
| 2283 | 0.192 | DDE1 | CTAAG |
| 2287 | 0.193 | FOK1 | GGATG |
| 2296 | 0.193 | MNL1 | CCTC |
| 2301 | 0.194 | ALU1 | AGCT |
| 2349 | 0.198 | BBV1 | GCTGC |
| 2349 | 0.198 | FNU4H1 | GCTGC |
| 2422 | 0.204 | HINF1 | GATTC |
| 2468 | 0.208 | HINF1 | GATTC |
| 2483 | 0.209 | BSTE11 | GGTAACC |
| 2503 | 0.211 | ALU1 | AGCT |
| 2524 | 0.212 | XBA1 | TCTAGA |
| 2534 | 0.213 | DDE1 | CTAAG |

18

FIG. 8(d)

| | | | |
|---|---|---|---|
| 2658 | 0.224 | RSA1 | GTAC |
| 2678 | 0.225 | SFNA1 | GCATC |
| 2726 | 0.230 | HINF1 | GAGTC |
| 2728 | 0.230 | HINC11 | GTCAAC |
| 2770 | 0.233 | HINF1 | GATTC |
| 2807 | 0.236 | HGA1 | GACGC |
| 2811 | 0.237 | DDE1 | CTTAG |
| 2965 | 0.250 | HINF1 | GATTC |
| 2984 | 0.251 | AVA11 | GGTCC |
| 2984 | 0.251 | SAU961 | GGTCC |
| 3012 | 0.254 | MNL1 | GAGG |
| 3024 | 0.255 | HINF1 | GATTC |
| 3032 | 0.255 | ALU1 | AGCT |
| 3048 | 0.257 | NDE1 | CATATG |
| 3090 | 0.260 | MNL1 | GAGG |
| 3093 | 0.260 | MBO11 | GAAGA |
| 3106 | 0.262 | RSA1 | GTAC |
| 3141 | 0.264 | TAQ1 | TCGA |
| 3168 | 0.267 | RSA1 | GTAC |
| 3193 | 0.269 | MBO1 | GATC |
| 3213 | 0.271 | HGIA1 | GTGCTC |
| 3216 | 0.271 | DDE1 | CTCAG |
| 3220 | 0.271 | MBO11 | GAAGA |
| 3234 | 0.272 | RSA1 | GTAC |
| 3263 | 0.275 | MNL1 | GAGG |
| 3333 | 0.281 | NDE1 | CATATG |
| 3412 | 0.287 | BCL1 | TGATCA |

| | | | |
|---|---|---|---|
| 3413 | 0.287 | MBO1 | GATC |
| 3415 | 0.288 | HPH1 | TCACC |
| 3457 | 0.291 | DDE1 | CTAAG |
| 3462 | 0.292 | HINF1 | GACTC |
| 3489 | 0.294 | TAQ1 | TCGA |
| 3522 | 0.297 | ECOR5 | GATATC |
| 3585 | 0.302 | RSA1 | GTAC |
| ➤ 3624 | 0.305 | BGL11 | AGATCT |
| 3625 | 0.305 | MBO1 | GATC |
| 3638 | 0.306 | MBO1 | GATC |
| 3689 | 0.311 | HPH1 | TCACC |
| 3792 | 0.319 | ALU1 | AGCT |

EP 0 107 278 B1

FIG. 8(e)

| | | | |
|---|---|---|---|
| 3847 | 0.324 | RSA1 | GTAC |
| 3905 | 0.329 | RSA1 | GTAC |
| 3970 | 0.334 | BSTN1 | CCAGG |
| 3970 | 0.334 | SCRF1 | CCAGG |
| 3979 | 0.335 | BSTE11 | GGTAACC |
| 4016 | 0.338 | MNL1 | GAGG |
| 4022 | 0.339 | SFNA1 | GCATC |
| 4025 | 0.339 | MBO11 | TCTTC |
| 4368 | 0.368 | HINF1 | GAGTC |
| 4384 | 0.369 | RSA1 | GTAC |
| 4410 | 0.371 | SFNA1 | GATGC |
| 4469 | 0.376 | SFNA1 | GATGC |
| 4520 | 0.381 | RSA1 | GTAC |
| 4523 | 0.381 | DDE1 | CTGAG |
| 4525 | 0.381 | MNL1 | GAGG |
| 4529 | 0.381 | ECOR5 | GATATC |
| 4533 | 0.382 | TAQ1 | TCGA |
| 4658 | 0.392 | HINF1 | GAATC |
| 4695 | 0.395 | ALU1 | AGCT |
| 4719 | 0.397 | XBA1 | TCTAGA |
| 4727 | 0.398 | SFNA1 | GCATC |
| → 4769 | 0.402 | ECOR1 | GAATTC |
| 4769 | 0.402 | XMN1 | GAATTCTTTC |
| 4778 | 0.402 | DDE1 | CTGAG |
| 4780 | 0.403 | HINF1 | GAGTC |
| 4848 | 0.408 | NDE1 | CATATG |
| 4961 | 0.418 | HINF1 | GATTC |
| 4988 | 0.420 | DDE1 | CTGAG |
| 5020 | 0.423 | ALU1 | AGCT |
| 5022 | 0.423 | DDE1 | CTGAG |
| 5049 | 0.425 | HINF1 | GATTC |
| 5053 | 0.426 | HPA11 | CCGG |
| 5085 | 0.428 | BCL1 | TGATCA |
| 5086 | 0.428 | MBO1 | GATC |
| → 5157 | 0.434 | PVU11 | CAGCTG |
| 5158 | 0.434 | ALU1 | AGCT |
| 5225 | 0.440 | ACC1 | GTAGAC |
| 5258 | 0.443 | PST1 | CTGCAG |
| 5285 | 0.445 | MNL1 | GAGG |
| 5339 | 0.450 | ECOR5 | GATATC |
| 5355 | 0.451 | RSA1 | GTAC |
| 5367 | 0.452 | HGIA1 | GTGCAC |
| 5394 | 0.454 | RSA1 | GTAC |
| 5402 | 0.455 | DDE1 | CTCAG |
| 5414 | 0.456 | BSTN1 | CCAGG |

20

FIG. 8(f)

| | | | |
|---|---|---|---|
| 5414 | 0.456 | SCRF1 | CCAGG |
| 5421 | 0.456 | MBO11 | GAAGA |
| 5451 | 0.459 | MBO1 | GATC |
| 5455 | 0.459 | ALU1 | AGCT |

.

.

| | | | |
|---|---|---|---|
| 5481 | 0.462 | FNU4H1 | GCGGC |
| 5490 | 0.462 | MNL1 | GAGG |
| 5560 | 0.468 | ALU1 | AGCT |
| 5562 | 0.468 | DDE1 | CTGAG |
| 5627 | 0.474 | XMN1 | GAAAGTATTC |
| 5653 | 0.476 | FOK1 | GGATG |
| 5657 | 0.476 | HINF1 | GAGTC |
| 5672 | 0.478 | PST1 | CTGCAG |
| 5674 | 0.478 | BBV1 | GCAGC |
| 5674 | 0.478 | FNU4H1 | GCAGC |
| 5754 | 0.485 | BSTN1 | CCTGG |
| 5754 | 0.485 | SCRF1 | CCTGG |
| 5761 | 0.485 | SAU961 | GGGCC |
| 5762 | 0.485 | HAE111 | GGCC |
| 5764 | 0.485 | BSTN1 | CCAGG |
| 5764 | 0.485 | SCRF1 | CCAGG |
| 5779 | 0.487 | MNL1 | CCTC |
| 5813 | 0.490 | ECOR5 | GATATC |
| 5821 | 0.490 | HAE111 | GGCC |
| 5844 | 0.492 | BBV1 | GCTGC |
| 5844 | 0.492 | FNU4H1 | GCTGC |
| 5845 | 0.492 | PST1 | CTGCAG |
| 5863 | 0.494 | BAL1 | TGGCCA |
| 5864 | 0.494 | HAE111 | GGCC |
| 5875 | 0.495 | SAU961 | GGGCC |
| 5876 | 0.495 | HAE111 | GGCC |
| 5886 | 0.496 | BAL1 | TGGCCA |
| 5887 | 0.496 | HAE111 | GGCC |
| 5898 | 0.497 | MNL1 | GAGG |
| 5899 | 0.497 | STU1 | AGGCCT |
| 5900 | 0.497 | HAE111 | GGCC |
| 5922 | 0.499 | ALU1 | AGCT |
| 5952 | 0.501 | MBO11 | GAAGA |
| 5955 | 0.501 | HINF1 | GAATC |
| 5961 | 0.502 | DDE1 | CTAAG |
| 5971 | 0.503 | SAU961 | GGGCC |

21

FIG. 8(g)

| | | | |
|---|---|---|---|
| 5972 | 0.503 | HAE111 | GGCC |
| 5987 | 0.504 | MB011 | TCTTC |
| 5994 | 0.505 | BSTN1 | CCTGG |
| 5994 | 0.505 | SCRF1 | CCTGG |
| 6000 | 0.505 | MB011 | TCTTC |
| 6021 | 0.507 | ALU1 | AGCT |
| 6026 | 0.507 | ACC1 | GTCTAC |
| 6037 | 0.508 | MNL1 | GAGG |
| 6121 | 0.515 | ALU1 | AGCT |
| 6139 | 0.517 | MB011 | TCTTC |
| 6177 | 0.520 | MNL1 | CCTC |
| 6211 | 0.523 | DDE1 | CTTAG |
| 6214 | 0.523 | ALU1 | AGCT |
| 6233 | 0.525 | HAE111 | GGCC |
| → 6248 | 0.526 | HIND111 | AAGCTT |
| 6249 | 0.526 | ALU1 | AGCT |
| 6275 | 0.528 | AVA11 | GGTCC |
| 6275 | 0.528 | SAU961 | GGTCC |
| 6305 | 0.531 | RSA1 | GTAC |
| 6361 | 0.536 | MB011 | TCTTC |
| 6379 | 0.537 | BBV1 | GCAGC |
| 6379 | 0.537 | FNU4H1 | GCAGC |
| → 6380 | 0.537 | PVU11 | CAGCTG |
| 6381 | 0.537 | ALU1 | AGCT |
| 6558 | 0.552 | AVA11 | GGTCC |

| | | | |
|---|---|---|---|
| 6558 | 0.552 | SAU961 | GGTCC |
| 6561 | 0.553 | BSTN1 | CCTGG |
| 6561 | 0.553 | SCRF1 | CCTGG |
| 6564 | 0.553 | HPH1 | GGTGA |
| 6629 | 0.558 | HINF1 | GAATC |
| 6639 | 0.559 | MB01 | GATC |
| 6674 | 0.562 | HINF1 | GAATC |
| 6677 | 0.562 | XBA1 | TCTAGA |
| 6683 | 0.563 | STU1 | AGGCCT |
| 6684 | 0.563 | HAE111 | GGCC |
| 6722 | 0.566 | BBV1 | GCAGC |
| 6722 | 0.566 | FNU4H1 | GCAGC |
| 6767 | 0.570 | SFNA1 | GCATC |
| 6793 | 0.572 | FOK1 | GGATG |
| 6848 | 0.577 | HINF1 | GACTC |

FIG. 8(h)

| | | | |
|---|---|---|---|
| 6874 | 0.579 | HINF1 | GATTC |
| 6911 | 0.582 | ECOR1 | GAATTC |
| 6916 | 0.582 | HPA11 | CCGG |
| 6984 | 0.588 | ALU1 | AGCT |
| 6991 | 0.589 | HINF1 | GACTC |
| 7028 | 0.592 | SAU961 | GGGCC |
| 7029 | 0.592 | HAE111 | GGCC |
| 7038 | 0.593 | DDE1 | CTCAG |
| 7052 | 0.594 | FOK1 | GGATG |
| 7056 | 0.594 | SAU961 | GGGCC |
| 7057 | 0.594 | HAE111 | GGCC |
| 7059 | 0.594 | MNL1 | CCTC |
| 7124 | 0.600 | MBO11 | TCTTC |
| 7155 | 0.603 | MBO11 | GAAGA |
| 7155 | 0.603 | XMN1 | GAAGAGTTTC |
| 7179 | 0.605 | DDE1 | CTAAG |
| 7182 | 0.605 | ALU1 | AGCT |
| 7185 | 0.605 | HPH1 | TCACC |
| 7194 | 0.606 | DDE1 | CTGAG |
| 7196 | 0.606 | MNL1 | GAGG |
| 7237 | 0.609 | ALU1 | AGCT |
| 7293 | 0.614 | AVA1 | CTCGGG |
| 7310 | 0.616 | MBO11 | GAAGA |
| 7313 | 0.616 | SFNA1 | GATGC |
| 7322 | 0.617 | BSTN1 | CCAGG |
| 7322 | 0.617 | SCRF1 | CCAGG |
| 7343 | 0.618 | RSA1 | GTAC |
| 7373 | 0.621 | HGIA1 | GAGCTC |
| 7373 | 0.621 | SAC1 | GAGCTC |
| 7374 | 0.621 | ALU1 | AGCT |
| 7376 | 0.621 | DDE1 | CTCAG |
| ➤7378 | 0.621 | PVU11 | CAGCTG |
| 7379 | 0.621 | ALU1 | AGCT |
| 7394 | 0.623 | HAE111 | GGCC |
| 7396 | 0.623 | BSTN1 | CCAGG |
| 7396 | 0.623 | SCRF1 | CCAGG |
| 7408 | 0.624 | DDE1 | CTGAG |
| 7410 | 0.624 | MNL1 | GAGG |
| 7438 | 0.626 | FOK1 | GGATG |
| 7485 | 0.630 | STU1 | AGGCCT |
| 7486 | 0.630 | HAE111 | GGCC |
| 7488 | 0.631 | MNL1 | CCTC |
| 7507 | 0.632 | HPH1 | GGTGA |
| 7516 | 0.633 | MNL1 | GAGG |
| 7529 | 0.634 | ALU1 | AGCT |
| 7547 | 0.636 | MBO11 | GAAGA |

EP 0 107 278 B1

FIG. 8(i)

| | | | |
|---|---|---|---|
| 7580 | 0.638 | HINF1 | GATTC |
| 7599 | 0.640 | HINC11 | GTCAAC |
| 7619 | 0.642 | MBO11 | GAAGA |
| 7634 | 0.643 | RSA1 | GTAC |
| 7637 | 0.643 | DDE1 | CTCAG |
| 7659 | 0.645 | ALU1 | AGCT |
| 7681 | 0.647 | HPH1 | GGTGA |
| 7705 | 0.649 | DDE1 | CTAAG |
| 7745 | 0.652 | HINF1 | GACTC |
| 7753 | 0.653 | MNL1 | GAGG |
| 7802 | 0.657 | HINF1 | GAGTC |
| 7809 | 0.658 | MBO1 | GATC |
| 7940 | 0.669 | BSTN1 | CCTGG |
| 7940 | 0.669 | SCRF1 | CCTGG |
| 7963 | 0.671 | MNL1 | CCTC |
| 7989 | 0.673 | ALU1 | AGCT |
| 8002 | 0.674 | HINF1 | GACTC |
| 8013 | 0.675 | HGIA1 | GTGCTC |
| 8021 | 0.675 | ALU1 | AGCT |
| 8031 | 0.676 | MNL1 | GAGG |
| 8035 | 0.677 | DDE1 | CTGAG |
| 8037 | 0.677 | MNL1 | GAGG |
| 8046 | 0.678 | HINF1 | GAATC |
| 8049 | 0.678 | HPH1 | TCACC |
| 8053 | 0.678 | DDE1 | CTGAG |
| 8058 | 0.679 | BSTN1 | CCTGG |
| 8058 | 0.679 | SCRF1 | CCTGG |
| 8067 | 0.679 | TAQ1 | TCGA |
| 8069 | 0.680 | MNL1 | GAGG |
| 8072 | 0.680 | BBV1 | GCTGC |
| 8072 | 0.680 | FNU4H1 | GCTGC |
| 8073 | 0.680 | PST1 | CTGCAG |
| 8086 | 0.681 | BCL1 | TGATCA |
| 8087 | 0.681 | MBO1 | GATC |
| 8109 | 0.683 | DDE1 | CTGAG |
| 8160 | 0.687 | HAE111 | GGCC |
| 8160 | 0.687 | SAU961 | GGCCC |
| 8190 | 0.690 | HPA11 | CCGG |

24

FIG. 8(j)

| | | | |
|---|---|---|---|
| 8190 | 0.690 | NCI1 | CCGGG |
| 8190 | 0.690 | SCRF1 | CCGGG |
| 8220 | 0.692 | RSA1 | GTAC |
| 8233 | 0.693 | AVA1 | CCCGGG |
| 8233 | 0.693 | NCI1 | CCCGG |
| 8233 | 0.693 | SCRF1 | CCCGG |
| 8233 | 0.693 | SMA1 | CCCGGG |
| 8234 | 0.693 | HPA11 | CCGG |
| 8234 | 0.693 | NCI1 | CCGGG |
| 8234 | 0.693 | SCRF1 | CCGGG |
| 8238 | 0.694 | HGIA1 | GTGCTC |
| 8243 | 0.694 | PST1 | CTGCAG |
| 8282 | 0.697 | NDE1 | CATATG |
| 8357 | 0.704 | DDE1 | CTTAG |
| 8366 | 0.705 | PVU11 | CAGCTG |
| 8367 | 0.705 | ALU1 | AGCT |
| 8376 | 0.705 | DDE1 | CTAAG |
| 8382 | 0.706 | HINF1 | GATTC |
| 8396 | 0.707 | MBO11 | GAAGA |
| 8410 | 0.708 | MNL1 | CCTC |
| 8417 | 0.709 | HAE111 | GGCC |
| 8417 | 0.709 | SAU961 | GGCCC |
| 8423 | 0.709 | MNL1 | CCTC |

| | | | |
|---|---|---|---|
| 8428 | 0.710 | BSTN1 | CCAGG |
| 8428 | 0.710 | SCRF1 | CCAGG |
| 8440 | 0.711 | BSTN1 | CCAGG |
| 8440 | 0.711 | SCRF1 | CCAGG |
| 8443 | 0.711 | FOK1 | GGATG |
| 8447 | 0.711 | AVA11 | GGTCC |
| 8447 | 0.711 | SAU961 | GGTCC |
| 8477 | 0.714 | BSTE11 | GGTAACC |
| 8492 | 0.715 | NDE1 | CATATG |
| 8643 | 0.728 | PST1 | CTGCAG |
| 9221 | 0.777 | MBO1 | GATC |
| 9263 | 0.780 | MNL1 | CCTC |
| 9266 | 0.780 | MNL1 | CCTC |
| 9294 | 0.783 | MNL1 | GAGG |
| 9335 | 0.786 | FOK1 | CATCC |
| 9350 | 0.787 | MBO11 | TCTTC |

FIG. 8(k)

| | | | |
|---|---|---|---|
| 9353 | 0.788 | MB011 | TCTTC |
| 9394 | 0.791 | BSTN1 | CCTGG |
| 9394 | 0.791 | SCRF1 | CCTGG |
| 9406 | 0.792 | MNL1 | CCTC |
| 9550 | 0.804 | MB01 | GATC |
| 9571 | 0.806 | MB011 | TCTTC |
| 9600 | 0.808 | HGIA1 | GTGCTC |
| 9603 | 0.809 | DDE1 | CTCAG |
| → 9614 | 0.810 | BAMH1 | GGATCC |
| 9615 | 0.810 | MB01 | GATC |
| 9626 | 0.811 | BSTN1 | CCAGG |
| 9626 | 0.811 | SCRF1 | CCAGG |
| 9641 | 0.812 | ALU1 | AGCT |
| 9643 | 0.812 | DDE1 | CTAAG |
| 9647 | 0.812 | MB011 | GAAGA |
| 9676 | 0.815 | HINF1 | GATTC |
| 9685 | 0.816 | MB01 | GATC |
| 9694 | 0.816 | FOK1 | CATCC |
| 9697 | 0.817 | BSTN1 | CCTGG |
| 9697 | 0.817 | SCRF1 | CCTGG |
| 9723 | 0.819 | MB011 | TCTTC |
| 9747 | 0.821 | NCI1 | CCCGG |
| 9747 | 0.821 | SCRF1 | CCCGG |
| 9748 | 0.821 | HPA11 | CCGG |
| 9762 | 0.822 | HAE11 | GGCGCC |
| 9762 | 0.822 | NAR1 | GGCGCC |
| 9763 | 0.822 | HHA1 | GCGC |
| 9777 | 0.823 | ALU1 | AGCT |
| 9787 | 0.824 | MNL1 | GAGG |
| 9791 | 0.825 | DDE1 | CTGAG |
| 9793 | 0.825 | MNL1 | GAGG |
| 9814 | 0.826 | HPA11 | CCGG |
| 9814 | 0.826 | NCI1 | CCGGG |
| 9814 | 0.826 | SCRF1 | CCGGG |
| 9819 | 0.827 | MNL1 | GAGG |
| 9826 | 0.828 | ALU1 | AGCT |
| 9843 | 0.829 | MB01 | GATC |
| 9864 | 0.831 | BSTN1 | CCTGG |
| 9864 | 0.831 | SCRF1 | CCTGG |
| 9881 | 0.832 | HINF1 | GACTC |
| 10246 | 0.863 | HINF1 | GATTC |
| 10279 | 0.866 | ALU1 | AGCT |
| 10281 | 0.866 | DDE1 | CTGAG |
| 10284 | 0.866 | ALU1 | AGCT |
| 10310 | 0.868 | TTH1111 | GACCCTGTC |

26

FIG. 8(L)

| | | | |
|---|---|---|---|
| 10336 | 0.870 | MNL1 | CCTC |
| 10347 | 0.871 | MNL1 | CCTC |
| 10351 | 0.872 | FOK1 | CATCC |
| 10455 | 0.880 | HINF1 | GAATC |
| 10463 | 0.881 | MNL1 | CCTC |
| 10473 | 0.882 | FOK1 | GGATG |
| 10477 | 0.882 | SAU961 | GGGCC |
| 10478 | 0.882 | HAE111 | GGCC |
| 10482 | 0.883 | ALU1 | AGCT |
| 10505 | 0.885 | PST1 | CTGCAG |
| 10512 | 0.885 | MNL1 | GAGG |
| 10536 | 0.887 | MBO1 | GATC |
| 10543 | 0.888 | PST1 | CTGCAG |
| 10545 | 0.888 | BBV1 | GCAGC |
| 10545 | 0.888 | FNU4H1 | GCAGC |
| 10563 | 0.890 | DDE1 | CTAAG |
| 10568 | 0.890 | SFNA1 | GCATC |
| 10589 | 0.892 | PVU11 | CAGCTG |
| 10590 | 0.892 | ALU1 | AGCT |
| 10605 | 0.893 | HPH1 | GGTGA |
| 10625 | 0.895 | ALU1 | AGCT |
| 10656 | 0.897 | HPH1 | TCACC |
| 10685 | 0.900 | SFNA1 | GATGC |
| 10693 | 0.901 | MBO11 | TCTTC |
| 10733 | 0.904 | BSTN1 | CCAGG |
| 10733 | 0.904 | SCRF1 | CCAGG |
| 10751 | 0.905 | BCL1 | TGATCA |
| 10752 | 0.905 | MBO1 | GATC |
| 10760 | 0.906 | HPH1 | GGTGA |
| 10763 | 0.906 | MBO11 | GAAGA |
| 10779 | 0.908 | MBO11 | GAAGA |
| 10865 | 0.915 | HPH1 | GGTGA |
| 10869 | 0.915 | ALU1 | AGCT |
| 10899 | 0.918 | MBO11 | GAAGA |
| 10925 | 0.920 | HPH1 | GGTGA |
| 10950 | 0.922 | HINF1 | GATTC |
| 10958 | 0.923 | MNL1 | CCTC |
| 11015 | 0.928 | BBV1 | GCAGC |

27

FIG. 8(m)

| 11015 | 0.928 | FNU4H1 | GCAGC |
|---|---|---|---|
| 11061 | 0.932 | HINC11 | GTTGAC |
| 11073 | 0.933 | ALU1 | AGCT |
| 11095 | 0.934 | FNU4H1 | GCGGC |
| 11132 | 0.938 | HPH1 | TCACC |
| 11135 | 0.938 | BSTN1 | CCTGG |
| 11135 | 0.938 | SCRF1 | CCTGG |
| 11137 | 0.938 | BAL1 | TGGCCA |
| 11138 | 0.938 | HAE111 | GGCC |
| 11145 | 0.939 | MBO1 | GATC |
| 11157 | 0.940 | DDE1 | CTAAG |
| 11170 | 0.941 | BAMH1 | GGATCC |
| 11171 | 0.941 | MBO1 | GATC |
| 11181 | 0.942 | ALU1 | AGCT |
| 11256 | 0.948 | BSTN1 | CCAGG |
| 11256 | 0.948 | SCRF1 | CCAGG |
| 11265 | 0.949 | HPH1 | TCACC |
| 11268 | 0.949 | MNL1 | CCTC |
| 11269 | 0.949 | DDE1 | CTCAG |
| 11272 | 0.949 | ALU1 | AGCT |
| 11278 | 0.950 | BSTN1 | CCAGG |
| 11278 | 0.950 | SCRF1 | CCAGG |
| 11300 | 0.952 | BBV1 | GCAGC |

| 11300 | 0.952 | FNU4H1 | GCAGC |
|---|---|---|---|
| 11303 | 0.952 | FNU4H1 | GCCGC |
| 11314 | 0.953 | NRU1 | TCGCGA |
| 11315 | 0.953 | FNUD11 | CGCG |
| 11324 | 0.954 | ALU1 | AGCT |
| 11330 | 0.954 | BSTN1 | CCAGG |
| 11330 | 0.954 | SCRF1 | CCAGG |
| 11349 | 0.956 | HPA11 | CCGG |
| 11356 | 0.956 | HAE11 | GGCGCT |
| 11357 | 0.956 | HHA1 | GCGC |
| 11367 | 0.957 | FOK1 | CATCC |
| 11381 | 0.958 | MNL1 | CCTC |
| 11428 | 0.962 | FNUD11 | CGCG |
| 11429 | 0.963 | HHA1 | GCGC |
| 11447 | 0.964 | HPA11 | CCGG |
| 11464 | 0.965 | MNL1 | GAGG |

28

FIG. 8(n)

| | | | |
|---|---|---|---|
| 11466 | 0.966 | HAE111 | GGCC |
| 11478 | 0.967 | MNL1 | GAGG |
| 11481 | 0.967 | RSA1 | GTAC |
| 11494 | 0.963 | MNL1 | CCTC |
| 11497 | 0.968 | BSTN1 | CCAGG |
| 11497 | 0.968 | SCRF1 | CCAGG |
| 1150C | 0.968 | HAE111 | GGCC |
| 11500 | 0.968 | SAU961 | GGCCC |
| 11504 | 0.969 | FNUD11 | CGCG |
| 11505 | 0.969 | HHA1 | GCGC |
| 11506 | 0.969 | FNUD11 | CGCG |
| 11515 | 0.970 | DDE1 | CTCAG |
| 11519 | 0.970 | HGIA1 | GAGCTC |
| 11519 | 0.970 | SAC1 | GAGCTC |
| 11520 | 0.970 | ALU1 | AGCT |
| 11533 | 0.971 | AVA1 | CTCGGG |
| 11557 | 0.973 | MBO11 | GAAGA |
| 11560 | 0.974 | XMN1 | GAAATACTTC |
| 11581 | 0.975 | MNL1 | GAGG |
| 11586 | 0.976 | ALU1 | AGCT |
| 11591 | 0.976 | MNL1 | GAGG |
| 11631 | 0.980 | DDE1 | CTTAG |
| 11648 | 0.981 | XBA1 | TCTAGA |
| 11652 | 0.981 | MNL1 | GAGG |
| 11701 | 0.985 | MBO11 | GAAGA |
| 11765 | 0.991 | ALU1 | AGCT |
| 11778 | 0.992 | ALU1 | AGCT |
| ➤11828 | 0.996 | HIND111 | AAGCTT |
| 11829 | 0.996 | ALU1 | AGCT |
| 11845 | 0.998 | BAMH1 | GGATCC |
| 11846 | 0.998 | MBO1 | GATC |
| ➤11868 | 0.999 | PVU11 | CAGCTG |
| 11869 | 1.000 | ALU1 | AGCT |

EP 0 107 278 B1

FIG. 9(a)

```
            -40                                                                      -20
    M  I  M  A  E  S  P  G  L  I  T  I  C  L  L  G  Y  L  L  S  A  E  C  T  V  F  L  D  H  E  N  A  N  K
TTTGCTAGCAGATTGTGAACATGATCATGGCAGAATCACCAGGCCTCATCACCATCTGCCTTTTAGGATATCTACTCAGTGCTGAATGTACAGTTTTTCTTGATCATGAAAACGCCAACA
     10      2C      30      40      50      60      70      80      90     t→100    110     120

        -4        -1    *                                                   20
    I  L  N  R  F  K  F  Y  N  S  G  K  L  E  E  F  V  D  G  N  L  E  R  E  C  M  E  E  K  C  S  F  E  E  A  R  E  V  F  E
AAATTCTGAATCGGCCAAAGAGGTATAATTCAGGTAAATTGGAAGAGTTTGTTCAAGGGAACCTTGAGAGAGAATGTATGGAAGAAAGTGTAGTTTTGAAGAAGCACGAGAAGTTTTTG
     13C     14C     150     160     170     180     190     200     210     22C     230     240

            40                                                              60
    N  T  E  R  T  T  E  F  M  K  Q  Y  V  C  G  D  R  C  E  S  N  P  C  L  N  G  G  S  C  K  D  D  I  N  S  Y  E  C  W  C
AAAACACTGAAAGAACAACTGAATTTTGGAAGCAGTATGTTGCTGGAGATCAGTGTGAGTCCAATCCATGTTTAAATGGCGGCAGTTGCAAGGATGACATTAATTCCTATGAATGTTGGT
     250  ←t  26C u→    270     290     290  ←u   v→     300     310     320     330     340     350     360

            80                                                          100
    P  F  G  F  E  G  K  N  C  E  L  E  V  T  C  N  I  K  N  G  R  C  E  Q  F  C  K  N  S  A  D  N  K  V  V  C  S  C  T  E
GTCCCTTTCGATTTGAAGGAAAGAACTGTGAATTAC ATGTAACATGTAACATTAAGAATGGCAGATGCGAGCAGTTTTGTAAAAATAGTGCTGATAACAAGGTGGTTTGCTCCTGTACTG
     370     38C     390 ←V   W→      410     420     430     440     450     460     470     480

            120                                                        140
    G  Y  R  L  A  E  N  Q  K  S  C  E  P  A  V  P  F  P  C  G  R  V  S  V  S  C  T  S  K  L  T  R  A  E  A  V  F  P  D  V
AGGGATATCGACTTGCAGAAAAACCAGAAGTCCTGTGAACCAGCAGTGCCATTTCCATGTGGAAGAGTTTCTGTTTCACAAACTTCTAAGCTCACCCGTGCTGAGGCTGTTTTTCCTGATG
     490     50C     510     520     530     540     550     560     570     580     590     600

            160                                                        180
    L  Y  V  N  S  T  E  A  E  T  I  L  D  N  I  T  L  S  T  Q  S  F  N  D  F  T  R  V  V  G  G  E  D  A  K  P  G  Q  F  P
TGGACTATGTAAATTCTACTGAAGCTGAAACCATTTTGGATAACATCACTCAAAGCACCCAATCATTTAATGACTTCACTCGGGGTTGTTGGTGGAGAAGATGCCAAACCAGGTCAATTCC
     610     62C     630     640     650     660     670     680     690     700     710     720

            200                                                        220
    W  Q  V  V  L  N  G  K  V  D  A  F  C  G  G  S  I  V  N  E  K  W  I  V  T  A  A  H  C  V  E  T  G  V  K  I  T  V  V  A
CTTGGCACGTTGTTTTGAATGGTAAAGTTGATGCATTCTGTGGAGGCTCTATCGTTAATGAAAAATGGATTGTAACTGCTGCCCACTGTGTTGAAACTGGTGTTAAAATTACAGTTGTCG
     730 ←X  74C y→    750     760     770     780     790     800     810     820     830     840

        240                                                        260
    C  E  H  K  R  N  I  E  E  T  E  H  T  E  Q  K  R  N  V  I  R  I  I  P  H  H  N  Y  N  A  A  I  N  K  Y  N  H  D  I  A  L  L
CAGCTGAACATAATATTGAGGAGACAGAACATACAGAGCAAAAGCGAAATGTGATTCGAATTATTCCTCACCACAACTACAATGCAGCTATTAATAAGTACAACCATGACATTGCCCTTC
     850     86C     870     980     890     900     910     920     930     940     950     960

        y z→    280                                                        300
    E  L  C  E  F  L  V  L  N  S  Y  V  V  T  F  I  C  I  A  D  K  E  Y  T  N  I  F  L  K  F  G  S  G  Y  V  S  G  W  G  R  V
TGGCACTGGACGAACCCTTAGTGCTAAACAGCTACGTTACACCTATTTGCATTGCTGACAAGGAATACACGAACATCTTCCTCAAATTTGGATCTGGCTATGTAAGTGGCTGGGGAAGAG
     970     93C     390     1000    1010    1020    1030    1040    1050    1060    1070    1080
```

30

FIG. 9(b)

```
          320                                                      340
   F  H  K  G  K  S  A  L  V  L  G  Y  L  R  V  P  L  V  D  R  A  T  C  L  R  S  T  K  F  T  I  Y  N  N  M  F  C  A  G  F
TCTTCCACAAAGGGAGATCAGCTTTAGTTCTTCAGTACCTTAGAGTTCCACTTGTTGACCGAGCCACATGTCTTCGATCTACAAAGTTCACCATCTATAACAACATGTTCTGTGCTGGCT
   1C90      110C     1110     1120     1130     1140     1150     1160     1170     1180     1190     1200

          360                                                      380
   H  E  G  G  K  D  S  C  Q  G  D  S  G  G  P  H  V  T  E  V  E  G  T  S  F  L  T  G  I  I  S  W  G  E  E  C  A  M  K  G
TCCATGAAGGAGCTAGAGATTCATGTCAAGGAGATAGTGGGGGACCCCATGTTACTGAAGTGGAAGGGACCAGTTTCTTAACTGGAATTATTAGCTGGGGTGAAGAGTGTGCAATGAAAG
   1210     122C     1230     1240     1250     1260     1270     1280     1290     1300     1310     1320

          400                                                      415
   K  Y  G  I  Y  T  K  V  S  R  Y  V  N  W  I  K  E  K  T  K  L  T  *
GCAAATATGGAATATATACCAAGGTATCCCGGTATGTCAACTGGATTAAGGAAAAAACAAAGCTCACTTAATGAAAGATGGATTTCCAAGGTTAATTCATTGGAATTGAAAATTAACAGG
   1330     134C     1350     1360     1370     1380     1390     1400     1410     1420     1430     1440

GCCTCTCACTAACTAATCACTTTCCCATCTTTTGTTAGATTTGAATATATACATTCTATGATCATTGCTTTTTCTCTTTACAGGGGAGAATTTCATATTTTACCTGAGCAAATTGATTAG
   1450     146C     1470     1480     1490     1500     1510     1520     1530     1540     1550     1560

AAAATGGAACCACTAGAGGAATATAATGTGTTAGGAAATTACAGTCATTTCTAAGGGCCCAGCCCTTGACAAAATTGTGAAGTTAAATTCTCCACTCTGTCCATCAGATACTATGGTTCT
   157C     158C     1590     1600     1610     1620     1630     1640     1650     1660     1670     1680

CCACTATGGCAACTAACTCACTCAATTTTCCCTCCTTAGCAGCATTCCATCTTCCCGATCTTCTTTGCTTCTCCAACCAAAACATCAATGTTTATTAGTTCTGTATACAGTACAGGATCT
   1690     170C     1710     1720     1730     1740     1750     1760     1770     1780     1790     1800

TTGGTCTACTCTATCACAACGCCAGTACCACACTCAATGAAGAAAGAACACAGGAGTAGCTGAGAGGCTAAAACTCATCAAAAACACTACTCCTTTTCCTCTACCCTATTCCTCAATCTTT
   1F10     182C     1830     1840     1850     1860     1870     1880     1890     1900     1910     1920

TACCTTTTCCAAATCCCAATCCCCAAATCAGTTTTTCTCTTTCTTACTCCCTCTCTCCCTTTTACCCTCCATGGTCGTTAAAGGAGAGATGGGGAGCATCATTCTGTTATACTTCTGTAC
   1930     194C     1950     1960     1970     1980     1990     2000     2010     2020     2030     2040

ACAGTTATACATGTCTATCAAACCCAGACTTGCTTCCATAGTGGGGACTTGCTTTTCAGAACATAGGGATGAAGTAAGGTGCCTGAAAAGTTTGGGGGAAAAGTTTCTTTCAGAGAGTTA
   2C50     206C     207C     2080     2090     2100     2110     2120     2130     2140     2150     2160
```

FIG. 9(c)

AGTTATTTTATATATATAATATATATATAAAATATATAATATACAATATAAATATATAGTGTGTGTGTGTATGCGTGTGTGTAGACACACACGCATACACACATATAATGGAAGCAATAA
2170    218C    219C    2200    2210    2220    2230    2240    2250    2260    2270    2280

GCCATTCTAAGAGCTTGTATGGTTATGGAGGTCTGACTAGGCATGATTTGACGAAGGCAAGATTGGCATATCATTGTAACTAAAAAAGCTGACATTGACCCAGACATATTGTACTCTTTC
2230    230C    2310    2320    2330    2340    2350    2360    2370    2380    2390    2400

TAAAAATAATAATAATAATGCTAACAGAAAGAAGACAACCGTTCGTTTGCAATCTACAGCTAGTAGAGACTTTGAGGAAGAATTCAACAGTGTGTCTTCAGCAGTGTTCAGAGCCAAGCA
2410    242C    2430    2440    2450    2460    2470    2480    2490    2500    2510    2520

AGAAGTTGAAGTTGCCTAGACCAGAGGACATAAGTATCATGTCTCCTTTAACTAGCATACCCCCAAGTGGAGAAGGGTGCAGCAGGCTCAAAGGCATAAGTCATTCCAATCAGCCAACTA
2530    254C    2550    2560    2570    2580    2590    2600    2610    2620    2630    2640

AGTTGTCCTTTTCTGGTTTCGTGTTCACCATGGAACATTTTGATTATAGTTAATCCTTCTATCTTGAATCTTCTAGAGAGTTGCTGACCAACTGACGTATGTTTCCCTTTGTGAATTAAT
2650    266C    2670    2680    2690    2700    2710    2720    2730    2740    2750    2760

AAACTGGTGTTCTGGTTCAAA
2770

```
                    BamHI  PvuII    HindIII

                             |
Oligo N3        5'     GATCCAGCTGA      3'
                          . . . . . . .
                          . . . . . . .

Oligo N4            3'     GTCGACTTCGA     5'
```

Fig. 10

```
Eco RI                         ClaI   HindIII PvuII BamHI
  |            10        20    |     30      | 40 |      50
5' GAA TTCTCATGTT TGACAGCTTA TCATCGATAA GCTTCAGCTG GATCCTCTAC
              60
    GCCGGACGCA   3'
```

Fig. 11

*Fig.12*

Fig.13